# EUROPÄISCHE PATENTSCHRIFT

(11) **EP 3 946 992 B1**
(45) Veröffentlichungstag und Bekanntmachung des Hinweises auf die Patenterteilung: **15.11.2023**
(21) Anmeldenummer: 20716716.4
(22) Anmeldetag: 25.03.2020
(51) Int. Cl.: B60H 3/02, F24F 13/28, B01D 53/62, B60H 3/06, B01D 53/04, B01D 53/047

(54) **VORRICHTUNG ZUM KOMBINIERTEN REDUZIEREN DES KOHLENDIOXID- UND WASSER- BZW. FEUCHTEGEHALTS, KRAFTFAHRZEUG UND VERFAHREN**
DEVICE FOR THE COMBINED REDUCTION OF THE CARBON DIOXIDE AND WATER OR MOISTURE CONTENT, MOTOR VEHICLE, AND METHOD
DISPOSITIF DE RÉDUCTION COMBINÉE DE LA TENEUR EN DIOXYDE DE CARBONE ET EN EAU OU EN HUMIDITÉ, VÉHICULE AUTOMOBILE ET PROCÉDÉ

(30) Priorität: 29.03.2019 DE 102019108348
(43) Veröffentlichungstag der Anmeldung: 09.02.2022
(73) Patentinhaber: MANN+HUMMEL GmbH, 71636 Ludwigsburg (DE)
(72) Erfinder: MBADINGA MOUANDA, Gelase, 74343 Sachsenheim (DE); JESSBERGER, Thomas, 71679 Asperg (DE); JANNER, Achim, 71638 Ludwigsburg (DE)
(74) Vertreter: Mann + Hummel Intellectual Property
(86) Internationale Anmeldenummer: PCT/EP2020/058334
(87) Internationale Veröffentlichungsnummer: WO 2020/200950

(56) Entgegenhaltungen:
- EP-A1- 1 226 860
- EP-B2- 1 226 860
- WO-A1-2004/101113
- WO-A1-2010/100739
- WO-A1-2012/084430

## Beschreibung

### Technisches Gebiet

Die vorliegende Erfindung betrifft eine Vorrichtung zum kombinierten Reduzieren des Kohlendioxid- und Wasser- bzw. Feuchtegehalts in einem umgrenzten Luftvolumen, insbesondere in einem Fahrgastraum eines Kraftfahrzeugs, ein Kraftfahrzeug mit einer derartigen Vorrichtung und ein Verfahren zum Betreiben einer derartigen Vorrichtung.

### Stand der Technik

Bei zumindest teilweise elektrisch angetriebenen Kraftfahrzeugen ist es zum Erreichen einer möglichst großen Reichweite zielführend, so viel Energie wie möglich einzusparen. Üblicherweise ist aus Komfortgründen ein Fahrgastraum eines derartigen Kraftfahrzeugs mit Hilfe einer Klimaanlage klimatisierbar. Hinsichtlich der erwünschten Energieersparnis ist es vorteilhaft, wenn die Klimaanlage zum Klimatisieren des Fahrgastraums möglichst wenig Frischluft aus der Umgebung des Kraftfahrzeugs ansaugt und erwärmt oder kühlt, sondern die sich im Fahrgastraum befindliche Luft im Umluftbetrieb umwälzt und klimatisiert.

Bei dem zuvor erwähnten Umluftbetrieb zum Klimatisieren des Fahrgastraums kann sich jedoch in der Ausatemluft von Insassen oder Passagieren enthaltenes Wasser in dem Fahrgastraum anreichern, was zu einem Beschlagen von Scheiben, beispielsweise einer Windschutzscheibe oder von Seitenscheiben, des Kraftfahrzeugs führen kann. Ursächlich hierfür ist, dass der entfeuchtende Effekt einer Klimaanlage, der sich durch eine Taupunktunterschreitung im Verdampfer-Wärmetauscher ergibt, bei Umluftbetrieb nicht genutzt werden kann. Dies gilt es im Hinblick auf Sicherheitsaspekte zu vermeiden oder zumindest zu reduzieren.

Des Weiteren kann sich in dem Fahrgastraum auch in der Ausatemluft enthaltenes Kohlendioxid anreichern. Dies kann zu Konzentrationsstörungen oder gar gesundheitlichen Beeinträchtigungen der Insassen führen. Auch dies gilt es sowohl im Hinblick auf Sicherheitsaspekte als auch im Hinblick auf Gesundheitsaspekte zu verhindern oder zumindest zu reduzieren, da schlimmstenfalls durch eine zu hohe Kohlendioxidkonzentration in der Innenraumluft die Konzentrationsfähigkeit des Fahrers derart stark herab gesetzt werden kann, dass Unfälle drohen.

Das Dokument WO 2004/101113 A1 zeigt eine Vorrichtung mit Sorptionseinheiten.

### Offenbarung der Erfindung

Es ist daher Aufgabe der Erfindung, eine verbesserte Vorrichtung zum Reduzieren des Kohlendioxid- und Wassergehalts für einen Fahrgastraum eines Kraftfahrzeugs zur Verfügung zu stellen.

Demgemäß wird eine Vorrichtung zum kombinierten Reduzieren des Kohlendioxid- und Wassergehalts in einem umgrenzten Luftvolumen, in einem Fahrgastraum eines Kraftfahrzeugs, vorgeschlagen. Die Vorrichtung gemäß Anspruch 1 umfasst eine erste Sorptionseinheit zum kombinierten Sorbieren von Kohlendioxid und Wasser, eine zweite Sorptionseinheit zum kombinierten Sorbieren von Kohlendioxid und Wasser, wobei die erste Sorptionseinheit und die zweite Sorptionseinheit jeweils mehrere Sorbenzien enthalten, wobei die Sorptionseinheiten jeweils von einem Sorptionsmodus, in dem die Sorptionseinheiten Kohlendioxid und Wasser aus Rohluft des umgrenzten Luftvolumens sorbieren, in einen Desorptionsmodus, in dem die Sorptionseinheiten Kohlendioxid und Wasser an zugeführte Regenerationsluft desorbieren, und umgekehrt verbringbar sind, und eine Luftverteilungseinrichtung, mit deren Hilfe die Sorptionseinheiten in Abhängigkeit von dem Kohlendioxid- und Wassergehalt in dem umgrenzten Luftvolumen jeweils von dem Sorptionsmodus in den Desorptionsmodus und umgekehrt derart wechselweise schaltbar sind, dass sich in zumindest einem Betriebszustand der Vorrichtung eine der beiden Sorptionseinheiten in dem Sorptionsmodus befindet, während sich die andere der beiden Sorptionseinheiten in dem Desorptionsmodus befindet.

Erfindungsgemäß ist vorgesehen, dass in dem Desorptionsmodus die durch die Sorptionseinheit, welche sich in dem Desorptionsmodus befindet, geleitete Regenerationsluft als beladene Regenerationsluft einer Umgebung zuführbar ist, insbesondere über eine Desorptionsleitung.

Das Kraftfahrzeug ist bevorzugt ein Elektrofahrzeug oder ein Hybridfahrzeug. Das Kraftfahrzeug kann jedoch auch mit Hilfe einer Brennkraftmaschine beziehungsweise eines Verbrennungsmotors angetrieben sein. Das Kraftfahrzeug umfasst insbesondere eine Karosserie, die das umgrenzte Luftvolumen umschließt. "Umgrenzt" heißt hierbei, dass die Karosserie die Grenzen beziehungsweise eine geometrische Ausdehnung des Luftvolumens definiert. Dies heißt insbesondere jedoch nicht zwingend, dass das umgrenzte Luftvolumen nicht im Luftaustausch mit einer Umgebung des Kraftfahrzeugs stehen kann. Das umgrenzte Luftvolumen ist bevorzugt der Fahrgastraum des Kraftfahrzeugs.

In dem umgrenzten Luftvolumen halten sich insbesondere Insassen oder Passagiere auf. Das umgrenzte Luftvolumen ist nicht zwingend einem Kraftfahrzeug zugeordnet. Das umgrenzte Luftvolumen kann auch einem Wasserfahrzeug, einer Baumaschine oder einem Baufahrzeug, einem Schienenfahrzeug, einer landwirtschaftlichen Maschine oder einem landwirtschaftlichen Fahrzeug oder einem Luftfahrzeug zugeordnet sein. Das umgrenzte Luftvolumen kann jedoch auch Teil eines Gebäudes oder einer stationären Maschine sein.

Insbesondere wird die Vorrichtung beziehungsweise die Luftverteilungseinrichtung auf Basis von Sensorsignalen angesteuert. Hierzu kann eine Steuereinheit vorgesehen sein. Ferner sind bevorzugt Sensoren, insbesondere Drucksensoren, Temperatursensoren und/oder Sensoren zum Erfassen des Kohlendioxid- und Wassergehalts vorgesehen. Beispielsweise wird der Sorptionsmodus aktiviert, wenn der Kohlendioxidgehalt über einen vorbestimmten Wert ansteigt.

Die Sorptionseinheiten sind geeignet, Kohlendioxid und Wasser zu adsorbieren. Die Sorptionseinheiten können auch geeignet sein, Kohlendioxid und Wasser zu absorbieren. Unter "Sorption" sind vorliegend Vorgänge zu verstehen, die zu einer Anreicherung eines Stoffes, beispielsweise von Kohlendioxid oder Wasser, innerhalb einer Phase oder auf einer Grenzfläche zwischen zwei Phasen führen. Die Anreicherung innerhalb einer Phase wird als Absorption bezeichnet, die Anreicherung an der Grenzfläche wird als Adsorption bezeichnet. Unter "Desorption" werden vorliegend Vorgänge verstanden, bei denen Atome oder Moleküle, insbesondere Kohlendioxid oder Wasser, die Oberfläche eines Festkörpers verlassen. Die Desorption stellt damit allgemein den Umkehrvorgang der Sorption dar.

Die Sorptionseinheiten können reine Adsorptionseinheiten sein oder als solche bezeichnet werden. Bevorzugt weisen die Sorptionseinheiten jeweils eine Kartuschenform auf, so dass diese einfach und schnell austauschbar sind. Die Sorptionseinheiten können eine zylinderförmige, insbesondere eine hohlzylinderförmige, eine plattenförmige, eine kuchenförmige oder eine beliebige andere Geometrie aufweisen.

Die Sorptionseinheiten umfassen jeweils mehrere Sorbenzien oder Sorptionsmittel. Es ist ein Sorbens vorgesehen, das geeignet ist, Kohlendioxid zu sorbieren, bevorzugt zu adsorbieren. Dieses Sorbens kann als Kohlendioxid-Sorbens oder CO2-Sorbens bezeichnet werden. Ein weiteres Sorbens ist vorgesehen, das geeignet ist, Wasser zu sorbieren, insbesondere zu adsorbieren. Dieses Sorbens kann als Wasser-Sorbens oder H2O-Sorbens bezeichnet werden. Die Sorbenzien können in Granulatform oder Faserform vorliegen, insbesondere in Form einer Schüttung. Insbesondere sind die Sorbenzien mit Hilfe eines Trägermaterials fixiert. Die Sorbenzien können auch reine Adsorbenzien sein oder als solche bezeichnet werden. Der Sorptionsmodus kann auch ein reiner Adsorptionsmodus sein oder als solcher bezeichnet werden. Der Desorptionsmodus kann auch als Regenerationsmodus bezeichnet werden. Darunter, dass die Sorptionseinheiten jeweils von dem Sorptionsmodus in den Desorptionsmodus und umgekehrt "verbringbar" sind, ist insbesondere zu verstehen, dass zwischen dem Sorptionsmodus und dem Desorptionsmodus hin und her geschaltet werden kann. Dieses Schalten erfolgt bevorzugt mit Hilfe der Luftverteilungseinrichtung, derart, dass den Sorptionseinheiten entweder die Rohluft oder die Regenerationsluft zugeführt wird. Hierzu weist die Luftverteilungseinrichtung bevorzugt mehrere Ventile oder Klappen auf. Die Luftverteilungseinrichtung kann ein Klappensystem oder eine Klappeneinrichtung sein oder als solche bezeichnet werden.

Darunter, dass die Luftverteilungseinrichtung geeignet ist, die Sorptionseinheiten "wechselweise" zu schalten ist insbesondere zu verstehen, dass sich beispielsweise die erste Sorptionseinheit im Sorptionsmodus befindet während sich die zweite Sorptionseinheit im Desorptionsmodus befindet. Nach dem Umschalten mit Hilfe der Luftverteilungseinrichtung verhält es sich genau umgekehrt.

Die Regenerationsluft wird erfindungsgemäß dem umgrenzten Luftvolumen oder der Umgebung des Kraftfahrzeugs entnommen.

Zunächst ist die Regenerationsluft unbeladen. Im Desorptionsmodus wird die unbeladene Regenerationsluft mit Kohlendioxid und Wasser beladen und als beladene Regenerationsluft der Umgebung zugeführt. Darunter, dass die Regenerationsluft mit Kohlendioxid und Wasser "unbeladen" ist, ist insbesondere zu verstehen, dass die Regenerationsluft in der sich im Desorptionsmodus befindenden Sorptionseinheit gespeichertes Kohlendioxid und Wasser aufnehmen kann. Dies schließt jedoch nicht aus, dass auch die unbeladene Regenerationsluft einen gewissen Gehalt an Kohlendioxid und Wasser enthalten kann. Allerdings ist die unbeladene Regenerationsluft nicht mit Kohlendioxid und Wasser gesättigt. Daher kann die unbeladene Regenerationsluft auch dem umgrenzten Luftvolumen entnommen werden.

Dadurch, dass die Sorptionseinheiten wechselweise betrieben werden, ist eine unterbrechungsfreie und damit kontinuierliche Reduktion des Kohlendioxid- und Wassergehalts in dem umgrenzten Luftvolumen möglich. Das heißt, in dem umgrenzten Luftvolumen kann ein Umluftbetrieb stattfinden ohne dass aus der Umgebung Umgebungsluft angesaugt werden muss, um den Kohlendioxid- und den Wassergehalt ausreichend niedrig zu halten. Somit kann das Anreichern von Kohlendioxid und Wasser in dem umgrenzten Luftvolumen mit den einleitend erläuterten Nachteilen zuverlässig verhindert werden. Durch den Verzicht auf das Zuführen von Umgebungsluft in das Fahrzeugklimatisierungssystem kann eine Energieersparnis erreicht werden, da auf das Kühlen oder Erwärmen von zugeführter Umgebungsluft verzichtet werden kann. Für den Fall, dass das Kraftfahrzeug elektrisch angetrieben ist, führt dies zu einer Verlängerung der Reichweite des Kraftfahrzeugs. Ferner kann durch den Verzicht auf das Zuführen von Umgebungsluft auch die Lebensdauer eines Innenraumfilters des Kraftfahrzeugs verlängert werden, da keine Schwebstoffe aus der Umgebungsluft herausgefiltert werden müssen. Als weitere vorteilhafte Wirkung ergibt sich, dass die Komponenten eines Fahrzeugklimatisierungssystems (jeweilige Wärmetauscher für Wärmesenke und Wärmequelle, Kompressor etc.) kleiner ausgelegt werden können, was Potential für ein Downsizing bietet.

In Ausführungsformen weist die Luftverteilungseinrichtung mehrere Ventile auf, die derart schaltbar sind, dass im Betrieb der Vorrichtung derjenigen Sorptionseinheit, die sich in dem Sorptionsmodus befindet, die Rohluft aus dem umgrenzten Luftvolumen zuführbar ist, um das Kohlendioxid und das Wasser aus der Rohluft zu entfernen, und derjenigen Sorptionseinheit, die sich in dem Desorptionsmodus befindet, die Regenerationsluft zuführbar ist, um das Kohlendioxid und das Wasser aus der Sorptionseinheit zu entfernen. Bevorzugt sind vier Ventile vorgesehen. Die Ventile können Klappenventile sein. Bevorzugt sind die Ventile Mehrwegeventile, insbesondere Dreiwegeventile oder 4,6,8-Wegeventile.

Erfindungsgemäß umfasst die Vorrichtung ferner zumindest ein Heizelement zum Einbringen von Wärme in diejenige Sorptionseinheit, die sich in dem Desorptionsmodus befindet. Bevorzugt ist jeder Sorptionseinheit ein derartiges Heizelement zugeordnet. Das Heizelement kann ein durch die jeweilige Sorptionseinheit geführter Heizdraht sein. Die Wärme kann jedoch auch in jeder anderen Art und Weise in die sich im Desorptionsmodus befindliche Sorptionseinheit eingebracht werden. Beispielsweise kann die Wärme Abwärme eines Elektromotors zum Antreiben des Kraftfahrzeugs sein. Die Heizung ist sinnvoller Weise nur dann eingeschaltet, wenn sich die jeweilige Sorptionseinheit in dem Desorptionsmodus befindet.

In Ausführungsformen ist in jede Sorptionseinheit ein Heizelement integriert. Das heißt, das Heizelement ist mit der diesem zugeordneten Sorptionseinheit fest, insbesondere untrennbar, verbunden. Dies kann beispielsweise mit Hilfe eines wie zuvor erwähnten Heizdrahts erfolgen. Insbesondere ist der ersten Sorptionseinheit ein erstes Heizelement und der zweiten Sorptionseinheit ist ein zweites Heizelement zugeordnet. Jede Sorptionseinheit kann mehrere Heizelemente umfassen.

Erfindungsgemäß ist das Heizelement stromaufwärts der Sorptionseinheiten positioniert ist. "Stromaufwärts" bedeutet vorliegend entlang einer Strömungsrichtung von den Sorptionseinheiten zugeführter unbeladener Regenerationsluft vor den Sorptionseinheiten. Das Heizelement ist in diesem Fall nicht in die Sorptionseinheiten integriert. Das Heizelement ist insbesondere außerhalb der Sorptionseinheiten angeordnet. Das Heizelement bringt Wärme in die unbeladene Regenerationsluft ein, welche im Desorptionsmodus der jeweiligen Sorptionseinheit Wasser und Kohlendioxid aufnimmt und als beladene Regenerationsluft von den Sorptionseinheiten weggeführt wird. Das Heizelement kann ein Wärmetauscher sein oder einen Wärmetauscher umfassen. Die zum Beheizen verwendete Wärme kann aus einem Kühlmittelkreislauf, beispielsweise eines konventionellen Verbrennungsmotors, einer Batteriekühlung oder eines Brennstoffzellenkühlkreises, entnommen werden. Zusätzlich können die Sorptionseinheiten jedoch auch noch in diese integrierte Heizelemente umfassen.

In Ausführungsformen weisen die Sorptionseinheiten ein gemeinsames Heizelement auf. Das heißt, es ist für beide Sorptionseinheiten nur ein oder genau ein Heizelement vorgesehen. Hierdurch kann auf ein separates Heizelement für jede Sorptionseinheit verzichtet werden.

In Ausführungsformen umfasst die Vorrichtung ferner ein Regenerationsventil, welches eine erste Schaltstellung, in welcher der sich im Desorptionsmodus befindlichen Sorptionseinheit die Regenerationsluft aus einer Umgebung des umgrenzten Luftvolumens zuführbar ist, eine zweite Schaltstellung, in welcher der sich im Desorptionsmodus befindlichen Sorptionseinheit die Regenerationsluft aus dem umgrenzten Luftvolumen zuführbar ist, und insbesondere eine dritte Schaltstellung aufweist, in welcher die sich im Desorptionsmodus befindliche Sorptionseinheit unter Vakuum regenerierbar ist. Das Regenerationsventil ist bevorzugt ein Dreiwegeventil. Stromabwärts des Regenerationsventils kann ein Rückschlagventil vorgesehen sein, das ein Zurückströmen der mit Kohlendioxid und Wasser beladenen Regenerationsluft in das umgrenzte Luftvolumen verhindert.

In Ausführungsformen umfasst die Vorrichtung ferner eine Gebläseeinrichtung, die derjenigen Sorptionseinheit, die sich im Sorptionsmodus befindet, die Rohluft zuführt. Die Gebläseeinrichtung ist bevorzugt ein Lüfter. Die Gebläseeinrichtung kann auch als erste Gebläseeinrichtung oder als Sorptionsgebläseeinrichtung bezeichnet werden. Eine Sorptionsleistung der sich im Sorptionsmodus befindlichen Sorptionseinheit kann beispielsweise durch eine Veränderung eines von der Gebläseeinrichtung erzeugten Volumenstroms angepasst werden. Der Volumenstrom kann mit Hilfe einer Drehzahländerung der Gebläseeinrichtung beeinflusst werden.

In Ausführungsformen ist die Gebläseeinrichtung Teil einer Klimaanlage. Hierdurch kann eine eigene Gebläseeinrichtung für die Vorrichtung eingespart werden. Insbesondere kann die Vorrichtung in die Klimaanlage teilintegriert oder vollintegriert sein. Alternativ kann die Vorrichtung als modulares Bauteil auch vollständig von der Klimaanlage getrennt und damit autark sein.

In Ausführungsformen umfasst die Vorrichtung ferner eine Gebläseeinrichtung, die derjenigen Sorptionseinheit, die sich in dem Desorptionsmodus befindet, die Regenerationsluft zuführt, wobei die Gebläseeinrichtung bezüglich der Sorptionseinheiten druckseitig oder saugseitig angeordnet ist. Die Gebläseeinrichtung ist bevorzugt ein Lüfter. Die Gebläseeinrichtung kann auch als zweite Gebläseeinrichtung oder als Desorptionsgebläseeinrichtung bezeichnet werden. Die erste Gebläseeinrichtung und die zweite Gebläseeinrichtung können ein gemeinsames Antriebselement umfassen, das mit Hilfe von Kupplungen mit Gebläserädern der Gebläseeinrichtungen koppelbar und von diesen entkoppelbar ist. Hierdurch kann ein Antriebselement eingespart werden. Das Antriebselement ist insbesondere ein Elektromotor.

In Ausführungsformen kann die Vorrichtung ferner eine Umgehungsleitung und ein Umgehungsventil umfassen, das wahlweise von einem ersten Schaltzustand, in welchem die Rohluft derjenigen Sorptionseinheit, welche sich in dem Sorptionsmodus befindet, zuführbar ist, in einen zweiten Schaltzustand, in welchem die Rohluft mit Hilfe der Umgehungsleitung um die Sorptionseinheiten herum zurück in das umgrenzte Luftvolumen leitbar ist, und umgekehrt schaltbar ist. Der zweite Schaltzustand (Bypass) wird bevorzugt dann gewählt, wenn der Kohlendioxid- und Wassergehalt unter einem vorbestimmten Wert liegt und somit keine Reduktion des Kohlendioxid- und Wassergehalts in dem umgrenzten Luftvolumen erforderlich ist. Alternativ hierzu kann die Umgehungsleitung auch aktiv geschaltet werden um Sensorprüfungen oder -kalibrierungen vorzunehmen und/oder wenn der Druckverlust über eine der Sorptionseinheiten einen vorbestimmten Grenzwert übersteigt.

Gemäß einer weiteren Ausführungsform können eine zweite Umgehungsleitung sowie ein zweites Umgehungsventil vorgesehen sein. Das zweite Umgehungsventil ist von einem ersten Schaltzustand, in welchem die Regenerationsluft derjenigen Sorptionseinheit, welche sich in dem Desorptionsmodus befindet, zuführbar ist, in einen zweiten Schaltzustand, in welchem die Regenerationsluft mit Hilfe der Umgehungsleitung unter Umgehung der Sorptionseinheiten in die Umgebung führbar ist, und umgekehrt schaltbar. Der zweite Schaltzustand, bei dem es sich um einen Desorptions-Bypass handelt, wird vorteilhaft eingesetzt, um ggf. im System vorgesehene Sensoren zu prüfen oder wenn der Druckverlust über eine der Sorptionseinheiten einen vorbestimmten Grenzwert übersteigt.

Eine noch weitere Ausführungsvariante sieht vor, der Desorptionsgebläseeinrichtung druckseitig nachgeschaltet einen Abzweig in der Desorptionsleitung vorzusehen, der mittels eines Rezirkulationsventils schaltbar ist. Der Abzweig mündet in eine Rezirkulationsleitung, die fluidisch mit einem Eingang des Regenerationsventils verbunden ist oder stromabwärts eines Abgangs des Regenerationsventils in eine an den Abgang angeschlossene Regenerationsluftleitung einmündet.

Hierdurch wird erreicht, dass zumindest ein Teil des von der Desorptionsgebläseeinrichtung geförderten Volumenstroms rezirkuliert, d. h. mehrmalig über die Sorptionseinheit, welche sich gerade im Desorptionsmodus befindet, geführt werden kann.

Dies hat in erster Linie energetische Vorteile, da die zur Regeneration eingebrachte Wärmemenge nicht nach einmaligem Durchgang durch die Sorptionseinheit, welche sich im Desorptionsmodus befindet in die Umgebung geführt wird, sondern so lange rezirkuliert (im Kreis geführt) wird, bis die Luft hinsichtlich CO2 und/oder Wassermenge vollständig beladen, d. h. gesättigt ist. Hierdurch kann folglich der Energieverbrauch der Heizeinrichtung drastisch reduziert werden, was einem energieeffizienten Betrieb der Gesamtvorrichtung weiter positiv beeinflusst.

Es kann gemäß einer Ausführungsform der Abzweig mittels eines Rezirkulationsventils schaltbar sein, sodass mittels der Desorptionsgebläseeinrichtung die Regerationsluft wahlweise durch die Sorptionseinheiten rezirkulierbar oder in die Umgebung leitbar ist.

Hinsichtlich der Rückführung in die Regenerationsluftleitung bestehen nun verschiedene Möglichkeiten.

Zunächst kann gemäß einer Ausführungsform die Rezirkulationsleitung fluidisch mit einem Eingang des Regenerationsventils verbunden sein, wobei mittels des Regenerationsventils eine vierte Schaltstellung, in welcher der sich im Desorptionsmodus befindlichen Sorptionseinheit die Regenerationsluft aus der Rezirkulationsleitung zuführbar ist, und umgekehrt schaltbar ist.

Gemäß dieser Ausführungsform erfolgt keine kontinuierliche Zuführung von unbeladener Regenerationsluft aus der Umgebung, sondern es ist vielmehr vorgesehen, das Regenerationsventil sowie das Rezirkulationsventil zyklisch paarweise zu schalten um eine Zuführung von unbeladener Frischluft und Abgabe von beladener Regenerationsluft aus dem "Rezirkulationskreislauf" zu ermöglichen.

Gemäß einer zweiten Ausführung, welche bevorzugt wird, kann vorgesehen sein, dass fluidisch zwischen dem Abgang des Regenerationsventils und der Einmündung der Rezirkulationsleitung in die Regenerationsluftleitung ein Drosselventil, insbesondere ein einstellbares Drosselventil angeordnet ist.

Gemäß dieser Ausführungsform kann nun eine kontinuierliche Zuführung von unbeladener Regenerationsluft aus der Umgebung erfolgen, wobei die Rate, mit der unbeladener Regenerationsluft aus der Umgebung dem "Rezirkulationskreislauf" zugeführt wird, nach Bedarf eingestellt werden kann.

In Ausführungsformen sind die Sorptionseinheiten dazu geeignet, die Rohluft neben Kohlendioxid und Wasser auch von Feinpartikeln, Stickoxiden und/oder flüchtigen organischen Verbindungen zu befreien. Hierzu können weitere geeignete Sorbenzien vorgesehen sein. Ferner können die Sorptionseinheiten zum Herausfiltern der Feinpartikel ein geeignetes Filtermedium umfassen. Das Filtermedium kann als Trägermaterial für die Sorbenzien fungieren.

In Ausführungsformen weisen die Sorptionseinheiten ein erstes Sorbens, das geeignet ist, Kohlendioxid zu adsorbieren, ein zweites Sorbens, das geeignet ist, Wasser zu adsorbieren und weitere Sorbenzien, die geeignet sind, die Rohluft von Feinpartikeln, Stickoxiden und/oder flüchtigen organischen Verbindungen zu befreien, auf, wobei die weiteren Sorbenzien zwischen zwei Trägerlagen, insbesondere aus einem Vlies, eingebracht sind, oder wobei die Sorbenzien und die weiteren Sorbenzien miteinander gemischt sind. Die Anzahl und Art der weiteren Sorbenzien ist beliebig. Die weiteren Sorbenzien können Aktivkohle umfassen, welche, insbesondere in Form einer Schüttung, bevorzugt zwischen die zwei Trägerlagen eingebracht ist. Das weitere Sorbens oder die weiteren Sorbenzien können mit dem CO2-Sorbens und/oder dem H2O-Sorbens vermischt sein, um eine oder mehrere gemischte Schüttungen aufzubauen.

In Ausführungsformen sind die Sorptionseinheiten dazu geeignet, die Rohluft neben Kohlendioxid und Wasser auch von Allergenen, Bakterien und/oder Viren zu befreien. Dies kann durch eine funktionelle Beschichtung von Trägermaterialien, beispielsweise von Vliesen, oder durch eine funktionelle Beschichtung der Sorbenzien erfolgen. Ferner können die Sorptionseinheiten unter einem Komfortaspekt zumindest eine Duftkomponente aufweisen.

Ferner wird ein Kraftfahrzeug gemäß Anspruch 17 mit einer derartigen Vorrichtung vorgeschlagen. Dabei ist die Vorrichtung auf Basis eines Belegungszustands des umgrenzten Luftvolumens mit Passagieren ansteuerbar, um den Kohlendioxid- und Wassergehalt in dem umgrenzten Luftvolumen unabhängig von dem Belegungszustand in einem vorgegebenen Toleranzfeld zu halten. Hierdurch ist stets gewährleistet, dass der Kohlendioxid- und Wassergehalt nicht unerwünscht ansteigt. Durch das Anpassen an den Belegungszustand kann Energie eingespart werden, da beispielsweise bei einem Belegungszustand mit einem Passagier eine geringere Förderleistung der ersten Gebläseeinrichtung gewählt wird als bei einem Belegungszustand mit vier Passagieren. Der Belegungszustand kann beispielsweise mit Hilfe von Gewichtssensoren oder optischen Sensoren erfasst werden.

Weiterhin wird ein Verfahren gemäß Anspruch 18 zum Betreiben einer derartigen Vorrichtung zum kombinierten Reduzieren des Kohlendioxid- und Wassergehalts in einem umgrenzten Luftvolumen, insbesondere in einem Fahrgastraum eines Kraftfahrzeugs, vorgeschlagen. Dabei umfasst die Vorrichtung eine erste Sorptionseinheit zum kombinierten Sorbieren von Kohlendioxid und Wasser, eine zweite Sorptionseinheit zum kombinierten Sorbieren von Kohlendioxid und Wasser und eine Luftverteilungseinrichtung zum wechselweisen Schalten der Sorptionseinheiten von einem Sorptionsmodus in einen Desorptionsmodus und umgekehrt. Das Verfahren umfasst die folgenden Schritte: a) Schalten einer der beiden Sorptionseinheiten in Abhängigkeit von dem Kohlendioxid- und Wassergehalt in dem umgrenzten Luftvolumen mit Hilfe der Luftverteilungseinrichtung in den Sorptionsmodus, in dem von der Sorptionseinheit Kohlendioxid und Wasser aus Rohluft des umgrenzten Luftvolumens sorbiert wird, b) Schalten der anderen der beiden Sorptionseinheiten mit Hilfe der Luftverteilungseinrichtung in den Desorptionsmodus, in dem von der Sorptionseinheit Kohlendioxid und Wasser an zugeführte Regenerationsluft desorbiert wird, und c) wechselweises Durchführen der Schritte a) und b) derart, dass eine der beiden Sorptionseinheiten in dem Sorptionsmodus betrieben wird, während die andere der beiden Sorptionseinheiten in dem Desorptionsmodus betrieben wird. Vorzugsweise werden die Schritte a) und b) gleichzeitig durchgeführt. Die für die Vorrichtung beschriebenen Merkmale und Ausführungsformen gelten auch für das Verfahren und umgekehrt.

Erfindungsgemäß wird in diejenige Sorptionseinheit, welche in dem Desorptionsmodus betrieben wird, Wärme eingebracht. Wie zuvor erwähnt, können hierfür Heizelemente vorgesehen sein. Die Wärme kann jedoch auch als Abwärme eines Elektromotors eingebracht werden.

In Ausführungsformen wird in dem umgrenzten Luftvolumen der Kohlendioxid- und Wassergehalt gemessen, um die Vorrichtung so anzusteuern, dass der Kohlendioxid- und Wassergehalt in dem umgrenzten Luftvolumen in einem vorgegebenen Toleranzfeld gehalten wird. Hierzu können in dem umgrenzten Luftvolumen ein Sensor oder mehrere Sensoren vorgesehen sein. Auch die Vorrichtung selbst umfasst bevorzugt eine Vielzahl unterschiedlicher Sensoren.

In Ausführungsformen wird ein Belegungszustands des umgrenzten Luftvolumens mit Passagieren erfasst, um die Vorrichtung so anzusteuern, dass der Kohlendioxid- und Wassergehalt in dem umgrenzten Luftvolumen unabhängig von dem Belegungszustand in dem vorgegebenen Toleranzfeld gehalten wird. Der Belegungszustand kann beispielsweise mit Hilfe von Gewichtssensoren oder optischen Sensoren erfasst werden. Durch die Ansteuerung basierend auf dem Belegungszustand kann Energie eingespart werden.

Erfindungsgemäß wird der Desorptionsmodus mit aus einer Umgebung des umgrenzten Luftvolumens entnommener Regenerationsluft, mit aus dem umgrenzten Luftvolumen entnommener Regenerationsluft oder unter Vakuum durchgeführt. Unter Vakuum kann der Desorptionsmodus bevorzugt bei niedrigeren Temperaturen durchgeführt werden. Dies ergibt vorteilhafterweise eine Energieersparnis. Für den Fall, dass die Regenerationsluft dem umgrenzten Luftvolumen entnommen wird, entspricht die Regenerationsluft insbesondere der Rohluft, die in diesem Fall nicht mit Kohlendioxid und Wasser gesättigt ist, so dass die als Regenerationsluft fungierende Rohluft noch Kohlendioxid und Wasser aufnehmen kann.

"Ein" ist vorliegend nicht zwangsweise als beschränkend auf genau ein Element zu verstehen. Vielmehr können auch mehrere Elemente, wie beispielsweise zwei, drei oder mehr, vorgesehen sein. Auch jedes andere hier verwendete Zählwort ist nicht dahingehend zu verstehen, dass eine genaue Beschränkung auf genau die entsprechende Anzahl von Elementen verwirklicht sein muss. Vielmehr sind zahlenmäßige Abweichungen nach oben und nach unten möglich.

### Kurze Beschreibung der Zeichnungen

Es zeigt dabei:
- Fig. 1:: eine schematische Ansicht einer Ausführungsform eines Kraftfahrzeugs;
- Fig. 2:: eine schematische Ansicht einer Ausführungsform einer Vorrichtung zur kombinierten Reduktion von Kohlendioxid und Wasser für das Fahrzeug;
- Fig. 3:: eine stark vereinfachte schematische Ansicht des Kraftfahrzeugs gemäß Fig. 1;
- Fig. 4:: eine weitere stark vereinfachte schematische Ansicht des Kraftfahrzeugs gemäß Fig. 1;
- Fig. 5:: eine weitere stark vereinfachte schematische Ansicht des Kraftfahrzeugs gemäß Fig. 1;
- Fig. 6:: eine weitere stark vereinfachte schematische Ansicht des Kraftfahrzeugs gemäß Fig. 1;
- Fig. 7:: eine weitere stark vereinfachte schematische Ansicht des Kraftfahrzeugs gemäß Fig. 1;
- Fig. 8:: eine weitere stark vereinfachte schematische Ansicht des Kraftfahrzeugs gemäß Fig. 1;
- Fig. 9:: eine weitere stark vereinfachte schematische Ansicht des Kraftfahrzeugs gemäß Fig. 1;
- Fig. 10:: eine stark vereinfachte schematische Ansicht einer weiteren Ausführungsform eines Kraftfahrzeugs;
- Fig. 11:: eine stark vereinfachte schematische Ansicht einer weiteren Ausführungsform eines Kraftfahrzeugs;
- Fig. 12:: eine stark vereinfachte schematische Ansicht einer weiteren Ausführungsform eines Kraftfahrzeugs;
- Fig. 13:: eine stark vereinfachte schematische Ansicht einer weiteren Ausführungsform eines Kraftfahrzeugs;
- Fig. 14:: eine schematische Ansicht einer Ausführungsform einer Gebläseeinrichtung für die Vorrichtung gemäß Fig. 2;
- Fig. 15:: eine schematische Ansicht einer weiteren Ausführungsform einer Gebläseeinrichtung für die Vorrichtung gemäß Fig. 2;
- Fig. 16:: eine schematische Ansicht einer Ausführungsform einer Kupplung für die Gebläseeinrichtung gemäß Fig. 14;
- Fig. 17:: eine schematische Ansicht einer weiteren Ausführungsform einer Gebläseeinrichtung für die Vorrichtung gemäß Fig. 2;
- Fig. 18:: eine schematische Ansicht einer Ausführungsform einer Kupplung für die Gebläseeinrichtung gemäß Fig. 17;
- Fig. 19:: ein schematisches Blockdiagramm einer Ausführungsform eines Verfahrens zum Betreiben der Vorrichtung gemäß Fig. 2;
- Fig. 20:: eine Weiterbildung des Kraftfahrzeugs gemäß Fig. 5 mit einer Rezirkulationsleitung im Desorptionskreis;
- Fig. 21:: eine Weiterbildung des Kraftfahrzeugs gemäß Fig. 5 mit einer gedrosselten Rezirkulationsleitung im Desorptionskreis;
- Fig. 22:: eine Weiterbildung des Kraftfahrzeugs gemäß Fig. 5 mit einer Umgehungsleitung im Desorptionskreis;
- Fig. 23:: eine stark vereinfachte schematische Ansicht einer weiteren Ausführungsform eines Kraftfahrzeugs; und
- Fig. 24:: eine weitere stark vereinfachte schematische Ansicht des Kraftfahrzeugs gemäß Fig. 23.

In den Figuren sind gleiche oder funktionsgleiche Elemente, sofern nichts anderes angegeben ist, mit denselben Bezugszeichen versehen worden.

### Ausführungsform(en) der Erfindung

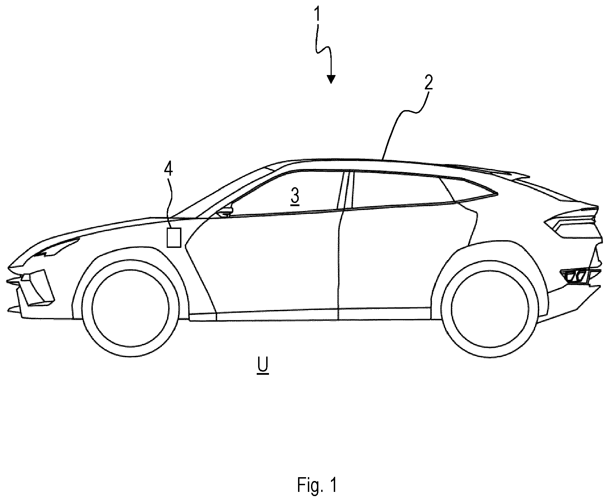

Die Fig. 1 zeigt eine schematische Ansicht einer Ausführungsform eines Kraftfahrzeugs 1. Das Kraftfahrzeug 1 ist bevorzugt ein Elektrofahrzeug oder ein Hybridfahrzeug. Das Kraftfahrzeug 1 kann jedoch auch mit Hilfe einer Brennkraftmaschine beziehungsweise eines Verbrennungsmotors angetrieben sein. Das Kraftfahrzeug 1 umfasst eine Karosserie 2, die ein umgrenztes Luftvolumen 3 umschließt. "Umgrenzt" heißt hierbei, dass die Karosserie 2 die Grenzen beziehungsweise eine geometrische Ausdehnung des umgrenzten Luftvolumens 3 definiert. Dies heißt jedoch nicht zwingend, dass das umgrenzte Luftvolumen 3 nicht im Luftaustausch mit einer Umgebung U des Kraftfahrzeugs 1 stehen kann.

Das umgrenzte Luftvolumen 3 ist ein Innenraum oder Fahrgastraum des Kraftfahrzeugs 1. Das umgrenzte Luftvolumen 3 kann jedoch auch einem Wasserfahrzeug, einer Baumaschine oder einem Baufahrzeug, einem Schienenfahrzeug, einer landwirtschaftlichen Maschine oder einem landwirtschaftlichen Fahrzeug oder einem Luftfahrzeug zugeordnet sein.

Nachfolgend wird jedoch davon ausgegangen, dass das umgrenzte Luftvolumen 3 der Fahrgastraum des Kraftfahrzeugs 1 ist. Daher wird das umgrenzte Luftvolumen 3 nachfolgend als Fahrgastraum bezeichnet. Der Fahrgastraum 3 ist mit Hilfe einer Klimaanlage 4 (Engl.: Heating, Ventilation, and Air Conditioning, HVAC) klimatisierbar. Zum Verlängern einer Reichweite eines derartigen elektrisch angetriebenen Kraftfahrzeugs 1 ist eine möglichst große Energieersparnis anzustreben. Mit Bezug auf die Klimaanlage 4 heißt dies, dass diese zum Klimatisieren des Fahrgastraums 3 möglichst wenig Frischluft aus der Umgebung U des Kraftfahrzeugs 1 ansaugen sollte.

Bei der Verwendung von in dem Fahrgastraum 3 aufgenommener Umluft zum Klimatisieren des Fahrgastraums 3 kann sich jedoch in der Ausatemluft von Insassen oder Passagieren enthaltenes Wasser (H2O) in dem Fahrgastraum 3 anreichern, was zu einem Beschlagen von Scheiben, beispielsweise einer Windschutzscheibe oder von Seitenscheiben, des Kraftfahrzeugs 1 führen kann. Dies gilt es im Hinblick auf Sicherheitsaspekte zu vermeiden oder zumindest zu reduzieren. Des Weiteren kann sich in dem Fahrgastraum 3 auch in der Ausatemluft enthaltenes Kohlendioxid (CO2) anreichern. Dies kann zu Konzentrationsstörungen oder gar gesundheitlichen Beeinträchtigungen der Insassen führen. Auch dies gilt es sowohl im Hinblick auf Sicherheitsaspekte als auch im Hinblick auf Gesundheitsaspekte zu verhindern oder zumindest zu reduzieren.

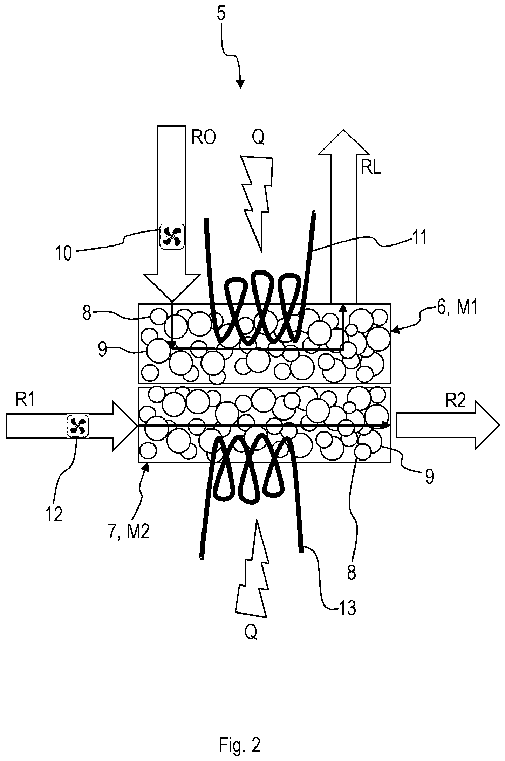

Die Fig. 2 zeigt eine schematische Ansicht einer Ausführungsform einer Vorrichtung 5 zur kombinierten Reduktion von CO2 und H2O, insbesondere Wasserdampf, innerhalb des Fahrgastraums 3. Mit Hilfe der Vorrichtung 5 können die zuvor erwähnten Nachteile verhindert oder deren Effekt zumindest reduziert werden. Darüber hinaus kann mit Hilfe der Vorrichtung 5 auch verhindert werden, dass Verunreinigungen aus der Umgebung U in den Fahrgastraum 3 gelangen, da durch die Aufbereitung der Umluft auf die Ansaugung von Umgebungsluft weitgehend verzichtet werden kann.

Die Vorrichtung 5 umfasst eine erste Sorptionseinheit 6 sowie eine zweite Sorptionseinheit 7. "Sorption" ist eine Sammelbezeichnung für Vorgänge, die zu einer Anreicherung eines Stoffes innerhalb einer Phase oder auf einer Grenzfläche zwischen zwei Phasen führen. Die Anreicherung innerhalb einer Phase nennt man genauer Absorption, die an der Grenzfläche Adsorption. Das heißt, die Sorptionseinheiten 6, 7 sind geeignet, Stoffe, wie beispielsweise CO2 und H2O, aber auch Stickoxide (NOX) und/oder flüchtige organische Verbindungen (Engl.: Volatile Organic Compounds, VOCs) zu Adsorbieren und/oder zu Absorbieren. Beispiele für flüchtige organische Verbindungen sind höhere Kohlenwasserstoffe. Die Sorptionseinheiten 6, 7 können auch geeignet sein, Schwefeldioxid (SO2) zu Adsorbieren und/oder zu Absorbieren. Bevorzugt sind die Sorptionseinheiten 6, 7 jedoch reine Adsorbereinheiten oder können als solche bezeichnet werden.

Die Sorptionseinheiten 6, 7 sind bevorzugt austauschbar und können, wie nachfolgend noch erläutert wird, wechselweise in einem Sorptionsmodus M1 und in einem Desorptionsmodus M2 betrieben werden. Die Sorptionseinheiten 6, 7 sind kartuschenförmig und können als Kartuschen oder Sorptionskartuschen bezeichnet werden. Der Sorptionsmodus M1 kann auch als Adsorptionsmodus bezeichnet werden. Der Desorptionsmodus M2 kann auch als Regenerationsmodus bezeichnet werden. Das heißt, die erste Sorptionseinheit 6 befindet sich in dem Sorptionsmodus M1 wenn sich die zweite Sorptionseinheit 7 in dem Desorptionsmodus M2 befindet und umgekehrt. Somit befinden sich nie beide Sorptionseinheiten 6, 7 gleichzeitig in demselben Modus M1, M2. Bevorzugt weisen die Sorptionseinheiten 6, 7 jeweils eine Kartuschenform auf, so dass diese einfach austauschbar sind.

Jede Sorptionseinheit 6, 7 umfasst ein erstes Sorbens 8 und ein zweites Sorbens 9.

Bevorzugt sind die Sorbenzien 8, 9 Adsorbenzien oder können als solche bezeichnet werden. Beispielsweise ist das erste Sorbens 8 geeignet, CO2 zu adsorbieren. Dementsprechend kann das zweite Sorbens 9 geeignet sein, H2O zu adsorbieren. Die beiden Sorbenzien 8, 9 können somit dem Fahrgastraum 3 H2O und CO2 entziehen. Das erste Sorbens 8 ist mit kleinen Kreisen illustriert. Das zweite Sorbens 9 ist mit großen Kreisen illustriert. Es können auch noch weitere Sorbenzien vorgesehen sein, die beispielsweise geeignet sind, NOX oder VOCs zu sorbieren. Es kann somit eine beliebige Anzahl unterschiedlicher Sorbenzien 8, 9 zur Aufbereitung der Umluft in dem Fahrgastraum 3 vorgesehen sein.

Beispielsweise sind neben den Sorbenzien 8, 9 zumindest ein weiteres Sorbens oder auch mehrere weitere Sorbenzien (nicht gezeigt) vorgesehen, die geeignet sind, die Rohluft RO von Feinpartikeln, NOx und/oder VOCs zu befreien. Die weiteren Sorbenzien können zwischen zwei Trägerlagen, insbesondere Trägerlagen aus einem Vlies, eingebracht sein. Alternativ können die Sorbenzien 8, 9 und die weiteren Sorbenzien miteinander gemischt sein. Die weiteren Sorbenzien können Aktivkohle umfassen, welche, insbesondere in Form einer Schüttung, bevorzugt zwischen die zwei Trägerlagen eingebracht ist. Das weitere Sorbens oder die weiteren Sorbenzien können mit dem ersten Sorbens 8 und/oder mit dem zweiten Sorbens 9 vermischt sein, um eine oder mehrere gemischte Schüttungen aufzubauen.

Weiterhin können die Sorptionseinheiten 6, 7 auch dazu geeignet sein, die Rohluft RO neben CO2 und H2O auch von Allergenen, Bakterien und/oder Viren zu befreien. Dies kann durch eine funktionelle Beschichtung von Trägermaterialien, beispielsweise von Vliesen, oder durch eine funktionelle Beschichtung der Sorbenzien 8, 9 erfolgen. Ferner können die Sorptionseinheiten 6, 7 unter einem Komfortaspekt zumindest eine Duftkomponente aufweisen.

Die Sorbenzien 8, 9 können jeweils in Form von kugelförmigem Granulat vorliegen. Bevorzugt sind die Sorbenzien 8, 9 auf einem Trägermaterial fixiert oder mit Hilfe eines Trägermaterials fixiert. Die Sorptionseinheiten 6, 7 können jeweils eine zylinderförmige, insbesondere eine hohlzylinderförmige, eine kuchenförmige oder eine rechteckförmige Geometrie aufweisen. Unter einer "kuchenförmigen" Geometrie ist vorliegend insbesondere eine flache kreiszylinderförmige Geometrie zu verstehen.

In der Fig. 2 befindet sich die erste Sorptionseinheit 6 in dem zuvor erwähnten Sorptionsmodus M1. Die zweite Sorptionseinheit 7 befindet sich in dem Desorptionsmodus M2. In dem Sorptionsmodus M1 wird der ersten Sorptionseinheit 6 mit CO2 und H2O beladene Rohluft RO aus dem Fahrgastraum 3 zugeführt. Hierzu kann eine erste Gebläseeinrichtung 10 vorgesehen sein. Die Rohluft RO wird zumindest abschnittsweise durch die erste Sorptionseinheit 6 hindurchgeleitet, wobei die Sorbenzien 8, 9 die Rohluft RO von CO2 und H2O reinigen. Die gereinigte Rohluft RO wird dem Fahrgastraum 3 als Reinluft RL wieder zugeführt.

Der ersten Sorptionseinheit 6 ist ein optionales erstes Heizelement 11 zugeordnet, mit dessen Hilfe den Sorbenzien 8, 9 Wärme Q zugeführt werden kann. Im Sorptionsmodus M1 ist das erste Heizelement 11 inaktiv, so dass dieses der ersten Sorptionseinheit 6 keine Wärme Q zuführt. Das erste Heizelement 11 kann ein durch die erste Sorptionseinheit 6 hindurchgeführter Heizdraht sein, der zum Einbringen der Wärme Q bestromt wird und so die Sorbenzien 8, 9 erhitzt. Die Wärme Q kann jedoch auch über jeden beliebigen anderen Weg eingebracht werden. Beispielsweise kann die Wärme Q auch Abwärme eines Elektromotors zum Antreiben des Kraftfahrzeugs 1 sein. Die Wärme Q kann auch Abwärme eines konventionellen Verbrennungsmotors, einer Batteriekühlung oder eines Brennstoffzellenkühlkreises sein. Das erste Heizelement 11 kann auch ein Wärmetauscher sein oder einen Wärmetauscher umfassen.

In dem Desorptionsmodus M2 wird der zweiten Sorptionseinheit 7 mit CO2 und H2O unbeladene Regenerationsluft R1 zugeführt. Hierzu kann eine zweite Gebläseeinrichtung 12 eingesetzt werden. Darunter, dass die unbeladene Regenerationsluft R1 mit CO2 und H2O "unbeladen" ist, ist zu verstehen, dass die unbeladene Regenerationsluft R1 in der zweiten Sorptionseinheit 7 gespeichertes CO2 und H2O aufnehmen kann. Das heißt, auch die unbeladene Regenerationsluft R1 kann einen gewissen Gehalt an CO2 und H2O aufweisen. Allerdings ist die unbeladene Regenerationsluft R1 nicht mit CO2 und H2O gesättigt. Die unbeladene Regenerationsluft R1 kann beispielsweise dem Fahrgastraum 3 oder der Umgebung U entnommen werden.

Ferner ist noch ein optionales zweites Heizelement 13 vorgesehen mit dessen Hife die zweite Sorptionseinheit 7 in dem Desorptionsmodus M2 beheizt wird und somit Wärme Q in die zweite Sorptionseinheit 7 eingebracht wird. Das erste Heizelement 11 und das zweite Heizelement 13 sind vorzugsweise identisch aufgebaut und werden wechselweise betrieben. Wie zuvor erwähnt, kann die Wärme Q beispielsweise auch in Form von Abwärme eines Elektromotors zugeführt werden. Die Wärme Q kann, wie ebenfalls zuvor erwähnt, jedoch auch Abwärme eines konventionellen Verbrennungsmotors, einer Batteriekühlung oder eines Brennstoffzellenkühlkreises sein. Das zweite Heizelement 13 kann auch ein Wärmetauscher sein oder einen Wärmetauscher umfassen.

Beim Erwärmen der Sorbenzien 8, 9 im Desorptionsmodus M2 der zweiten Sorptionseinheit 7 geben diese CO2 und H2O an die unbeladene Regenerationsluft R1 ab. Das heißt, CO2 und H2O werden desorbiert. Zur Desorption ist bevorzugt eine Temperatur von über 55 °C erforderlich. Die unbeladene Regenerationsluft R1 wird durch die zweite Sorptionseinheit 7 hindurchgeführt, nimmt dort CO2 und H2O auf und wird von der zweiten Sorptionseinheit 7 als beladene Regenerationsluft R2 abgeführt. Insbesondere wird die beladene Regenerationsluft R2 der Umgebung U zugeführt.

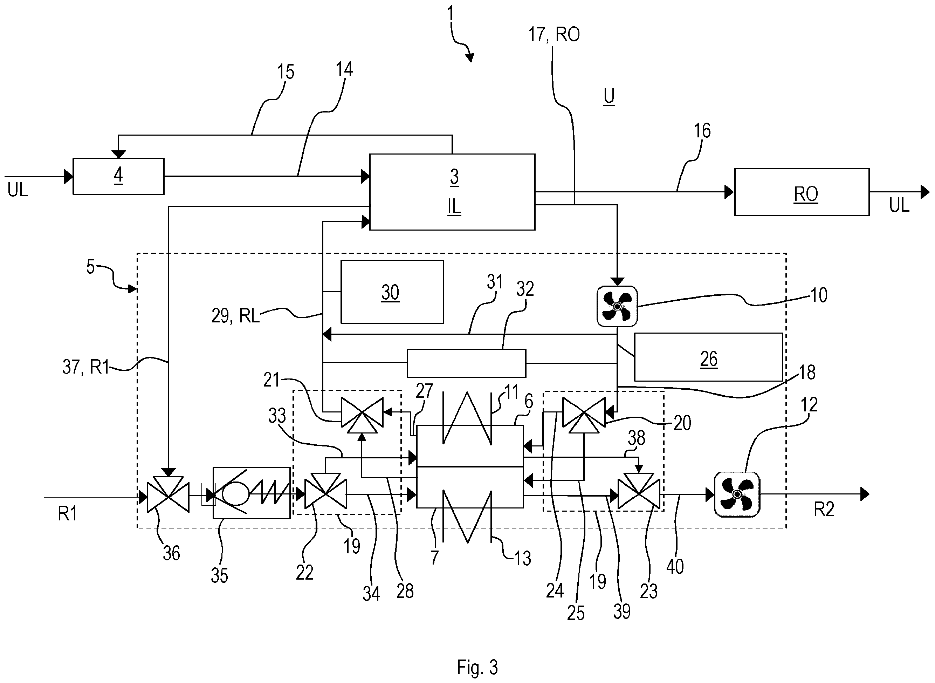

Die Fig. 3 zeigt eine stark schematisierte Ansicht des Kraftfahrzeugs 1 mit der Vorrichtung 5. Bei dieser Ausführungsform des Kraftfahrzeugs 1 arbeitet die Vorrichtung 5 unabhängig von der Klimaanlage 4. Dem Fahrgastraum 3 kann über die Klimaanlage 4 Umgebungsluft UL zugeführt werden. Die Umgebungsluft UL kann mit Hilfe der Klimaanlage 4 temperiert und von Schwebstoffen, wie beispielsweise Staub oder Pollen, gereinigt werden. Hierzu kann die Klimaanlage 4 einen Innenraumfilter umfassen. Die gereinigte und temperierte Umgebungsluft UL wird dem Fahrgastraum 3 über eine Leitung 14 als Innenraumluft IL zugeführt. Über eine Leitung 15 kann die Innenraumluft IL wieder der Klimaanlage 4 zugeführt werden, beispielsweise um die Innenraumluft IL im Umluftbetrieb zu erwärmen oder zu kühlen.

Im Betrieb des Kraftfahrzeugs 1 belädt sich die Innenraumluft IL in dem Fahrgastraum 3 mit CO2 und H2O. Die beladene Innenraumluft IL ist die zuvor erwähnte Rohluft RO. Diese wie zuvor erwähnte Rohluft RO kann über eine Leitung 16 zumindest teilweise der Umgebung U zugeführt werden. Darüber hinaus kann die mit CO2 und H2O beladene Rohluft RO mit Hilfe einer Leitung 17 zumindest teilweise der Vorrichtung 5 zugeführt werden. Insbesondere führt die Leitung 17 die Rohluft RO der ersten Gebläseeinrichtung 10 zu. Die erste Gebläseeinrichtung 10 ist dabei druckseitig montiert. Die erste Gebläseeinrichtung 10 kann jedoch auch saugseitig montiert werden.

Stromabwärts der ersten Gebläseeinrichtung 10 ist eine Leitung 18 vorgesehen, welche die Rohluft RO einer Luftverteilungseinrichtung 19 zuführt. Die Luftverteilungseinrichtung 19 umfasst mehrere Ventile 20 bis 23, insbesondere Dreiwegeventile. Die Ventile 20 bis 23 sind vorzugsweise als Klappenventile ausgebildet. Dementsprechend kann die Luftverteilungseinrichtung 19 auch als Klappeneinrichtung oder Klappensystem bezeichnet werden.

Die Leitung 18 ist mit dem Ventil 20 in Fluidverbindung, das wiederum über Leitungen 24, 25 mit den beiden Sorptionseinheiten 6, 7 in Fluidverbindung ist. Je nach Schaltstellung des Ventils 20 kann die Rohluft RO wahlweise entweder der ersten Sorptionseinheit 6 oder der zweiten Sorptionseinheit 7 zugeführt werden. In oder an der Leitung 18 kann ein Sensor 26, insbesondere ein CO2-, H20- und/oder Temperatursensor, vorgesehen sein. Insbesondere verbindet die Leitung 24 das Ventil 20 mit der ersten Sorptionseinheit 6. Die Leitung 25 verbindet das Ventil 20 mit der zweiten Sorptionseinheit 7.

Stromabwärts der Sorptionseinheiten 6, 7 ist ein Ventil 21 der Luftverteilungseinrichtung 19 vorgesehen. Das Ventil 21 ist mit Hilfe einer Leitung 27 mit der ersten Sorptionseinheit 6 und mit Hilfe einer Leitung 28 mit der zweiten Sorptionseinheit 7 verbunden. Eine Leitung 29 verbindet das Ventil 21 mit dem Fahrgastraum 3. Auch die Leitung 29 kann einen Sensor 30, insbesondere einen CO2-, H20- und/oder Temperatursensor, umfassen. Mit Hilfe des Sensors 30 kann beispielsweise erfasst werden, wenn die sich im Sorptionsmodus M1 befindliche Sorptionseinheit 6, 7 erschöpft ist. Zwischen den Leitungen 18, 29 ist eine Umgehungsleitung 31 zum Umgehen der Sorptionseinheiten 6, 7 vorgesehen. Ferner ist zwischen den Leitungen 18, 29 auch ein Sensor 32, insbesondere ein Drucksensor, vorgesehen, der eine Druckdifferenz zwischen den Leitungen 18, 29 erfassen kann.

Die Luftverteilungseinrichtung 19 umfasst ein weiteres Ventil 22, welches mit Hilfe einer Leitung 33 mit der ersten Sorptionseinheit 6 und mit Hilfe einer Leitung 34 mit der zweiten Sorptionseinheit 7 verbunden ist. Dem Ventil 22 ist ein Rückschlagventil 35 sowie ein Regenerationsventil 36 vorgeschaltet. Das Regenerationsventil 36 ist vorzugsweise ein Dreiwegeventil. Dem Regenerationsventil 36 wird die Umgebungsluft UL als unbeladene Regenerationsluft R1 zugeführt. Des Weiteren kann dem Regenerationsventil 36 auch über eine Leitung 37 die Innenraumluft IL als unbeladene Regenerationsluft R1 zugeführt werden.

Stromabwärts der Sorptionseinheiten 6, 7 ist ein weiteres Ventil 23 der Luftverteilungseinrichtung 19 positioniert. Das Ventil 23 ist mit Hilfe einer Leitung 38 mit der ersten Sorptionseinheit 6 und mit Hilfe einer Leitung 39 mit der zweiten Sorptionseinheit 7 verbunden. Eine Leitung 40 verbindet das Ventil 23 mit der zweiten Gebläseeinrichtung 12. Die zweite Gebläseeinrichtung 12 ist dabei saugseitig, das heißt stromabwärts der Sorptionseinheiten 6, 7 positioniert. Die zweite Gebläseeinrichtung 12 kann jedoch auch druckseitig, das heißt stromaufwärts der Sorptionseinheiten 6, 7 positioniert sein. Die zweite Gebläseeinrichtung 12 gibt die beladene Regeneratiosluft R2 an die Umgebung U ab.

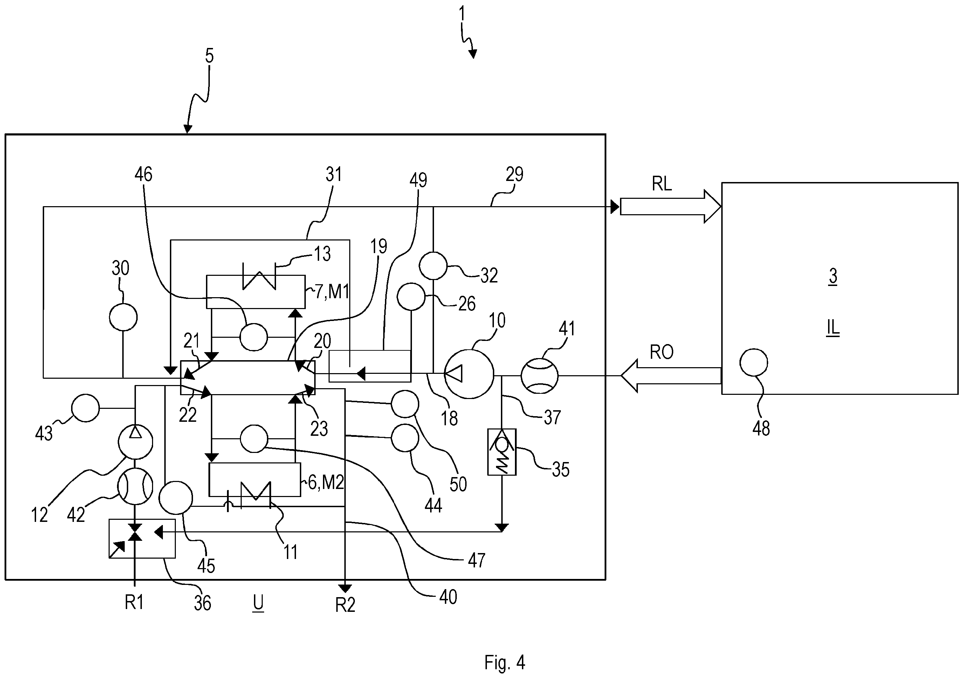

Die Fig. 4 zeigt das Kraftfahrzeug 1 mit einem Betriebszustand der Vorrichtung 5, bei dem sich die erste Sorptionseinheit 6 im Desorptionsmodus M2 und sich die zweite Sorptionseinheit 7 im Sorptiosmodus M1 befindet. Anders als in der Fig. 3 sind beide Gebläseeinrichtungen 10, 12 druckseitig positioniert. Der ersten Gebläseeinrichtung 10 kann ein Volumenstromsensor 41 vorgeschaltet sein. Stromabwärts des Regenerationsventils 36 ist ebenfalls ein Volumenstromsensor 42 vorgesehen.

Stromabwärts der zweiten Gebläseeinrichtung 12 ist ein weiterer Sensor 43, insbesondere ein CO2-, H20- und/oder Temperatursensor, vorgesehen. Ferner ist auch in oder an der Leitung 40 ein weiterer Sensor 44, insbesondere ein CO2-, H20- und/oder Temperatursensor, vorgesehen. Ferner ist ein Sensor 45 vorgesehen, der geeignet ist, eine Druckdifferenz zwischen der Leitung 40 und der Luftverteilungseinrichtung 19 zu erfassen. Jeder Sorptionseinheit 6, 7 ist ein Sensor 46, 47, insbesondere ein Differenzdrucksensor, zugeordnet. Auch in dem Fahrgastraum 3 kann ein Sensor 48, insbesondere ein CO2-, H20- und/oder Temperatursensor, vorgesehen sein. Um die Umgehungsleitung 31 zu aktivieren und zu deaktivieren, ist ein Umgehungsventil 49 vorgesehen. Ferner umfasst die Leitung 40 einen Drucksensor 50, insbesondere zum Bestimmen des Absolutdrucks.

In der Fig. 4 ist das Regenerationsventil 36 so geschaltet, dass die unbeladene Regenerationsluft R1 aus der Umgebung U und nicht aus dem Fahrgastraum 3 angesaugt wird. Die Ventile 22, 23 sind so geschaltet, dass die unbeladene Regenerationsluft R1 mit Hilfe der druckseitig angeordneten zweiten Gebläseeinrichtung 12 durch die erste Sorptionseinheit 6 gedrückt wird. Dabei ist das erste Heizelement 11 in Betrieb. Die erste Sorptionseinheit 6 gibt CO2 und H2O an die unbeladene Regenerationsluft R1 ab, die dann als beladene Regenerationsluft R2 der Umgebung U zugeführt wird.

Gleichzeitig sind die Ventile 20, 21 so geschaltet, dass mit Hilfe der ersten Gebläseeinrichtung 10 mit CO2 und H2O beladene Rohluft RO aus dem Fahrgastraum 3 angesaugt wird und durch die zweite Sorptionseinheit 7 geleitet wird. In der zweiten Sorptionseinheit 7 werden das CO2 und das H2O adsorbiert und die unbeladene Reinluft RL wird wieder dem Fahrgastraum 3 zugeführt. Das Umgehungsventil 49 ist so geschaltet, dass die Umgehungsleitung 31 inaktiv ist.

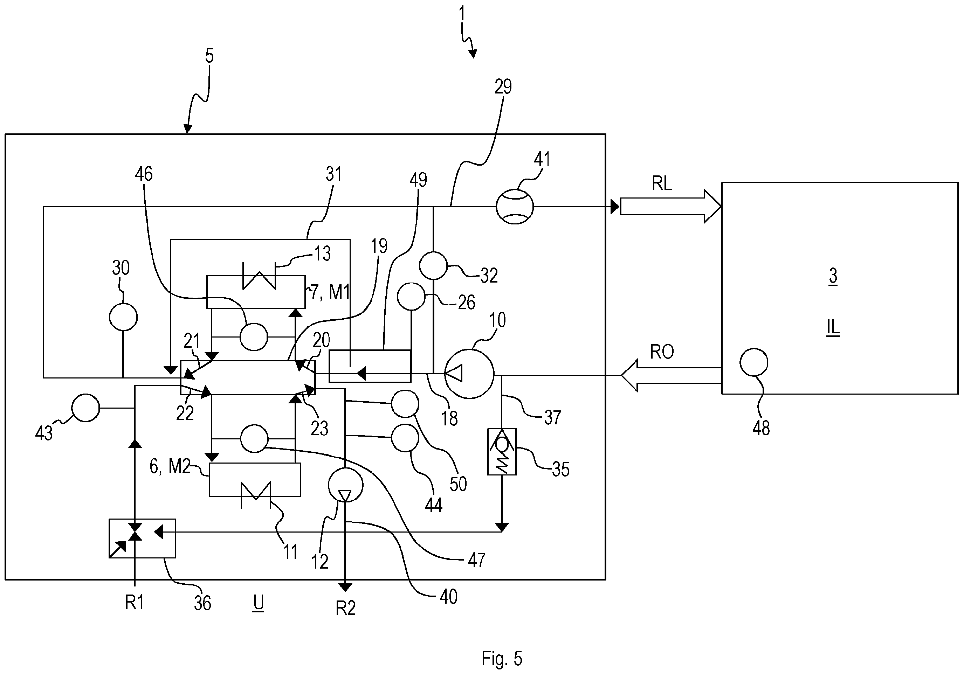

Die Fig. 5 zeigt das Kraftfahrzeug 1 mit einer Weiterbildung der Vorrichtung 5. Dabei befindet sich in der Fig. 5 die erste Sorptionseinheit 6 im Desorptionsmodus M2 und die zweite Sorptionseinheit 7 im Sorptionsmodus M1. Die Vorrichtung 5 gemäß der Fig. 5 unterscheidet sich von der Vorrichtung 5 gemäß der Fig. 4 im Wesentlichen dadurch, dass die zweite Gebläseeinrichtung 12 nicht druckseitig sondern saugseitig positioniert ist. "Saugseitig" heißt hierbei stromabwärts der Sorptionseinheiten 6, 7.

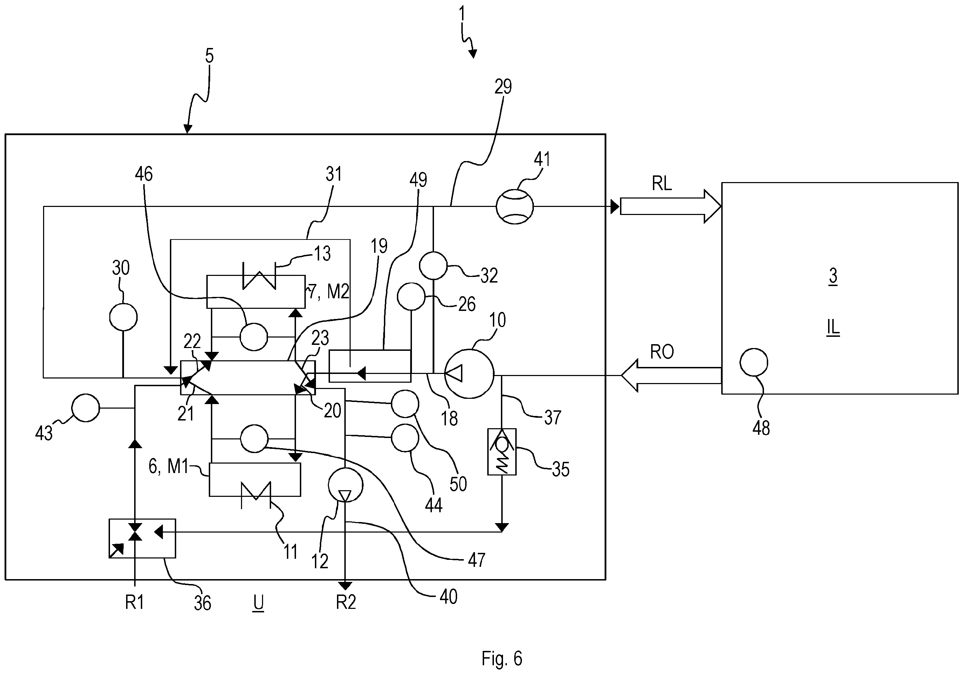

Die Fig. 6 zeigt die Vorrichtung 5 gemäß Fig. 5, wobei sich die erste Sorptionseinheit 6 im Sorptionsmodus M1 und die zweite Sorptionseinheit 7 im Desorptionsmodus M2 befindet. Das Regenerationsventil 36 ist nach wie vor so geschaltet, dass die unbeladene Regenerationsluft R1 aus der Umgebung U angesaugt wird. Die Ventile 20, 21 der Luftverteilungseinrichtung 19 sind so geschaltet, dass die mit Hilfe der ersten Gebläseeinrichtung 10 aus dem Fahrgastraum 3 angesaugte und mit CO2 und H2O angereicherte Rohluft RO durch die erste Sorptionseinheit 6 hindurchgeleitet und als von CO2 und H2O gereinigte Reinluft RL wieder dem Fahrgastraum 3 zugeführt wird. Die Umgehungsleitung 31 ist inaktiv.

Die Ventile 22, 23 sind so geschaltet, dass die aus der Umgebung U angesaugte unbeladene Regenerationsluft R1 mit Hilfe der zweiten Gebläseeinrichtung 12 durch die zweite Sorptionseinheit 7 gesaugt wird. Das zweite Heizelement 13 ist dabei in Betrieb. Die zweite Gebläseeinrichtung 12 bläst die mit CO2 und H2O angereicherte beladene Regenerationsluft R2 in die Umgebung U.

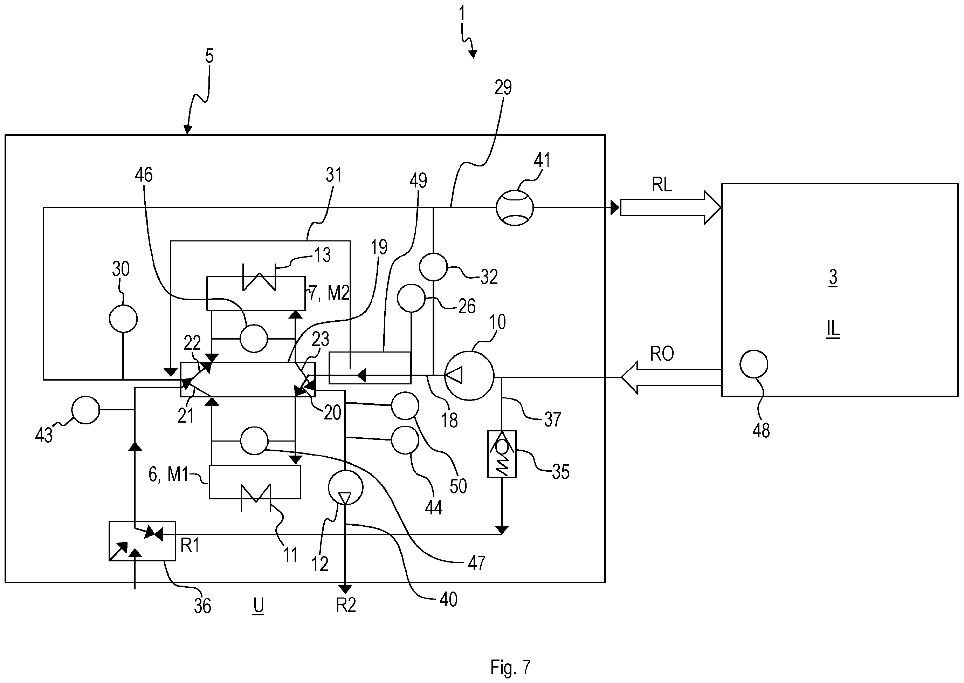

Die Fig. 7 zeigt wiederum die Vorrichtung 5 gemäß den Fig. 5 und 6. Dabei befindet sich die erste Sorptionseinheit 6 im Sorptionsmodus M1 und die zweite Sorptionseinheit 7 Desorptionsmodus M2. Im Unterschied zu Fig. 6 ist jedoch das Regenerationsventil 36 so geschaltet, dass als unbeladene Regenerationsluft R1 die Rohluft RO aus dem Fahrgastraum 3 verwendet wird. Das heißt, die unbeladene Regenerationsluft R1 wird nicht aus der Umgebung U angesaugt.

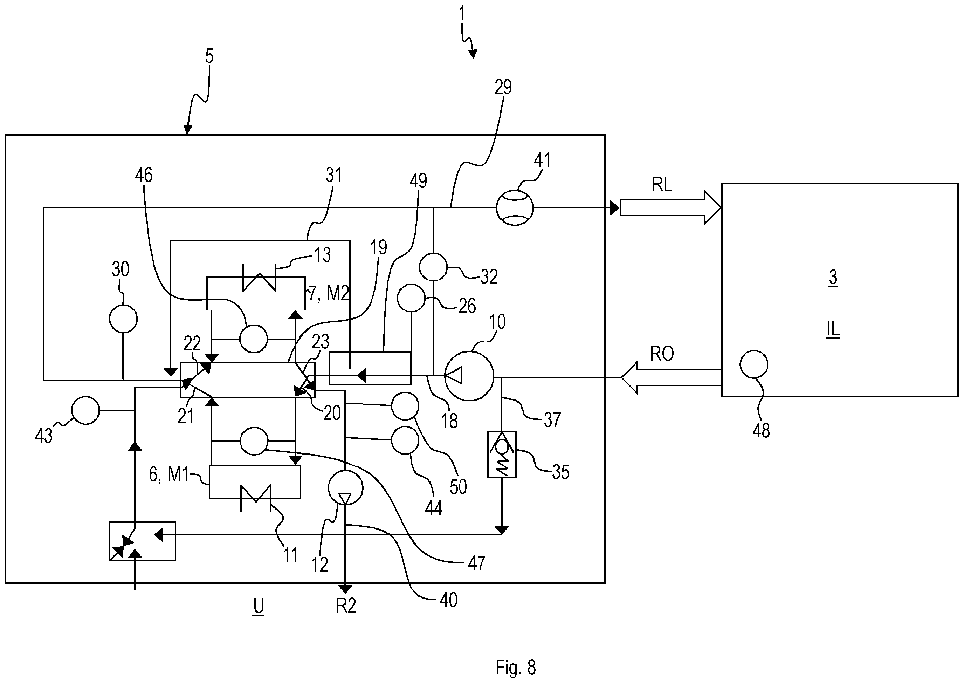

Die Fig. 8 zeigt wiederum die Vorrichtung 5 gemäß den Fig. 5 und 6. Dabei befindet sich die erste Sorptionseinheit 6 im Sorptionsmodus M1 und die zweite Sorptionseinheit 7 Desorptionsmodus M2. Im Unterschied zu Fig. 6 ist jedoch das Regenerationsventil 36 so geschaltet, dass weder aus der Umgebung U noch aus dem Fahrgastraum 3 unbeladene Regenerationsluft R1 angesaugt wird. Vielmehr erfolgt der Desorptionsmodus M2 der zweiten Sorptionseinheit 7 unter Vakuum oder Unterdruck, das oder der mit Hilfe der zweiten Gebläseeinrichtung 12 erzeugt wird. Die Umgehungsleitung 31 ist dabei inaktiv.

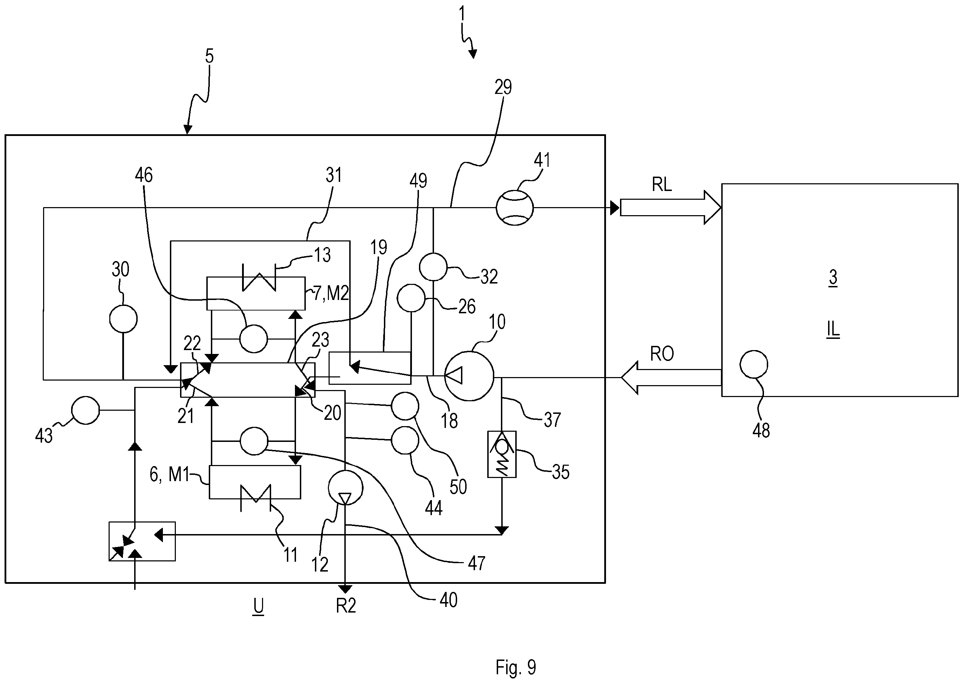

Die Fig. 9 zeigt wiederum die Vorrichtung 5 gemäß den Fig. 5 und 6. Dabei befindet sich die erste Sorptionseinheit 6 im Sorptionsmodus M1 und die zweite Sorptionseinheit 7 Desorptionsmodus M2. Im Unterschied zu Fig. 8 ist jedoch das Umgehungsventil 49 so geschaltet, dass die Umgehungsleitung 31 aktiv ist. Das heißt, die Rohluft RO wird aus dem Fahrgastraum 3 um die Sorptionseinheiten 6, 7 herumgeführt. Die Luft der Umgehungsleitung 31 wird durch die Leitung der Reinnluft RL in den Fahrgastraum zurückgespeist. Die zweite Sorptionseinheit 7 befindet sich im Desorptionsmodus M2 unter Vakuum oder Unterdruck, wie zuvor schon mit Bezug auf Fig. 8 erläutert.

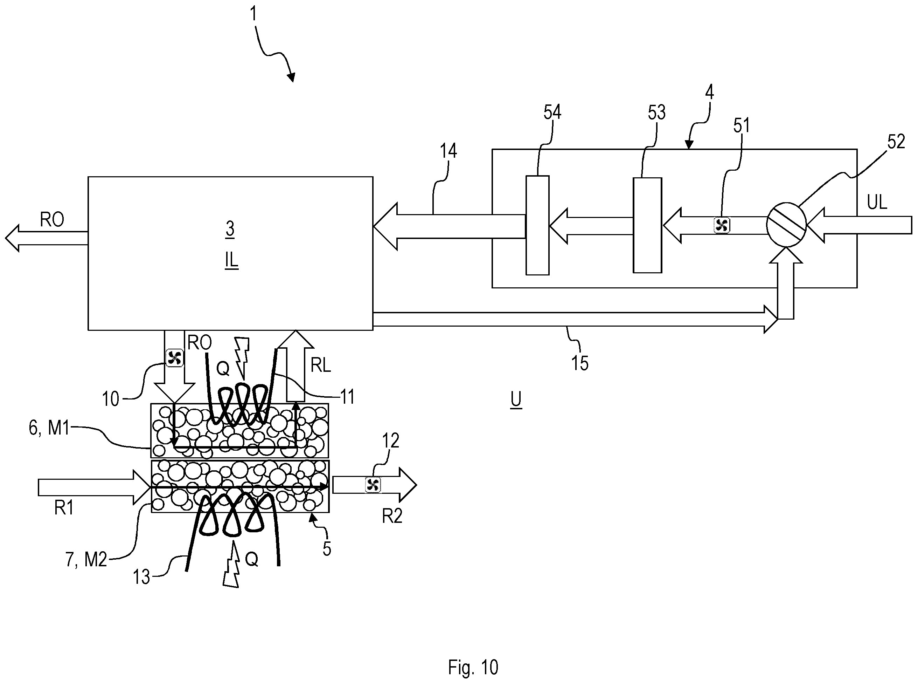

Die Fig. 10 zeigt eine Weiterbildung des Kraftfahrzeugs 1, bei der die Vorrichtung 5 unabhängig von der Klimaanlage 4 operiert und somit nicht in diese integriert ist. Die Vorrichtung 5 arbeitet somit autark und kann beispielsweise als autarkes Modul in dem Kraftfahrzeug 1 verbaut werden. Zwischen der Vorrichtung 5 und der Klimaanlage 4 ist keine direkte Schnittstelle vorgesehen. Die Klimaanlage 4 umfasst eine eigene Gebläseeinrichtung 51, die geeignet ist, Umgebungsluft UL aus der Umgebung oder Innenraumluft IL aus dem Fahrgastraum 3 anzusaugen, zu temperieren und/oder zu reinigen.

Weiterhin weist die Klimaanlage 4 ein Ventil 52, beispielsweise ein Klappenventil, auf, das so geschaltet werden kann, dass entweder Umgebungsluft UL aus der Umgebung U oder Innenraumluft IL aus dem Fahrgastraum 3 angesaugt wird. Zum Temperieren sind ein Kühlelement 53 und ein Heizelement 54 vorgesehen. Ferner kann die Klimaanlage 4 auch noch ein nicht gezeigtes Filterelement, beispielsweise in Form eines Innenraumfilters, aufweisen.

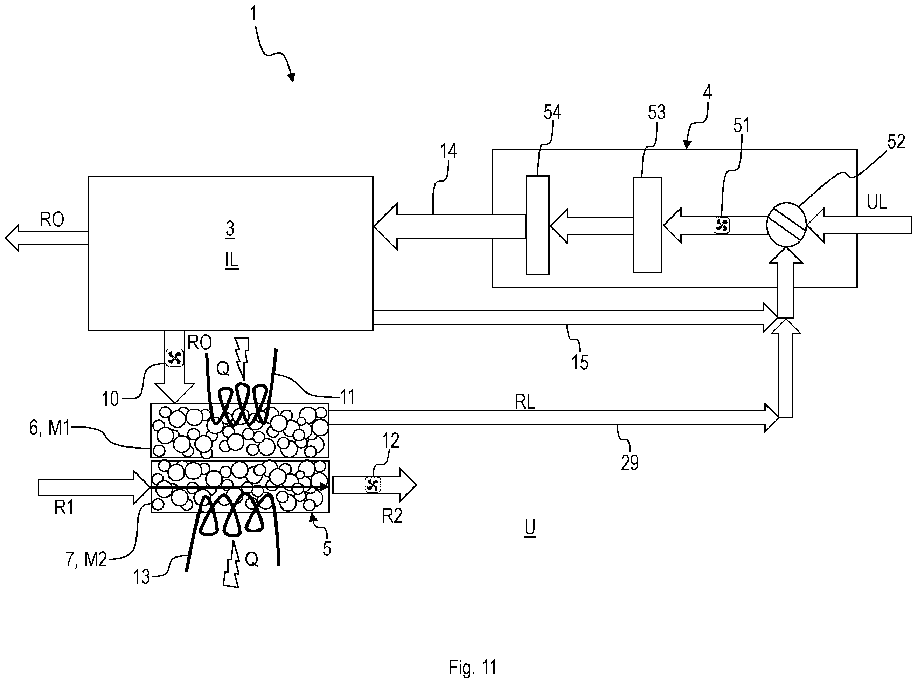

Die Fig. 11 zeigt eine Weiterbildung des Kraftfahrzeugs 1, bei der die Vorrichtung 5 in die Klimaanlage 4 teilintegriert ist. Hierbei ist die Leitung 29, welche die Reinluft RL von den Sorptionseinheiten 6, 7 wegführt, nicht direkt mit dem Fahrgastraum 3 sondern mit der Klimaanlage 4, insbesondere mit der Leitung 15, die dem Umluftbetrieb der Klimaanlage 4 dient, verbunden. Hierdurch ist eine sehr einfache Schnittstelle zwischen der Vorrichtung 5 und der Klimaanlage 4 möglich.

Das heißt insbesondere, dass die Reinluft RL nicht direkt in den Fahrgastraum 3, sondern über die Klimaanlage 4 in diesen geleitet wird. Hierdurch ist es beispielsweise möglich, die von CO2 und H2O befreite Reinluft RL zu temperieren. Das heißt, ein Teil der Innenraumluft IL wird als Rohluft RO durch die erste Sorptionseinheit 6 geleitet und ein weiterer Teil der Innenraumluft IL wird im Umluftbetrieb durch die Klimaanlage 4 geleitet. Sowohl die Vorrichtung 5 als auch die Klimaanlage 4 weisen eine eigene Gebläseeinrichtung 10, 12, 51 auf, die jedoch synchronisiert werden können.

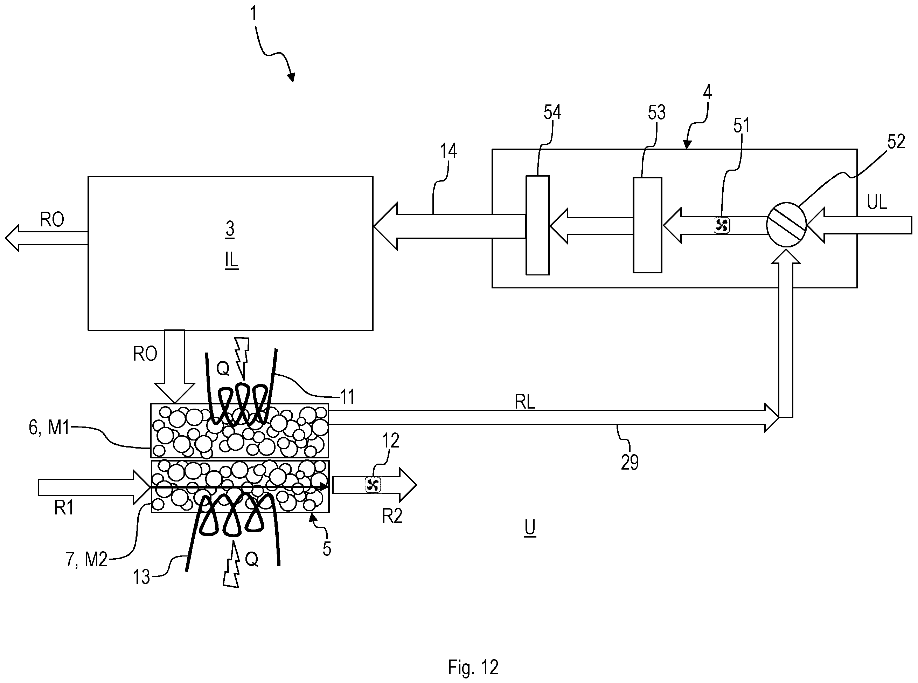

Die Fig. 12 zeigt eine Weiterbildung des Kraftfahrzeugs 1, bei der die Vorrichtung 5 in die Klimaanlage 4 vollintegriert ist. Bei dieser Ausführungsform der Vorrichtung 5 ist die erste Gebläseeinrichtung 10 verzichtbar, da im Sorptionsmodus M1 die Gebläseeinrichtung 51 der Klimaanlage 4 zum Durchleiten der Rohluft RO durch die entsprechende Sorptionseinheit 6, 7 genutzt wird. Hierzu ist die Leitung 29, welche die Reinluft RL von den Sorptionseinheiten 6, 7 wegführt, direkt mit dem Ventil 52 der Klimaanlage 4 verbunden.

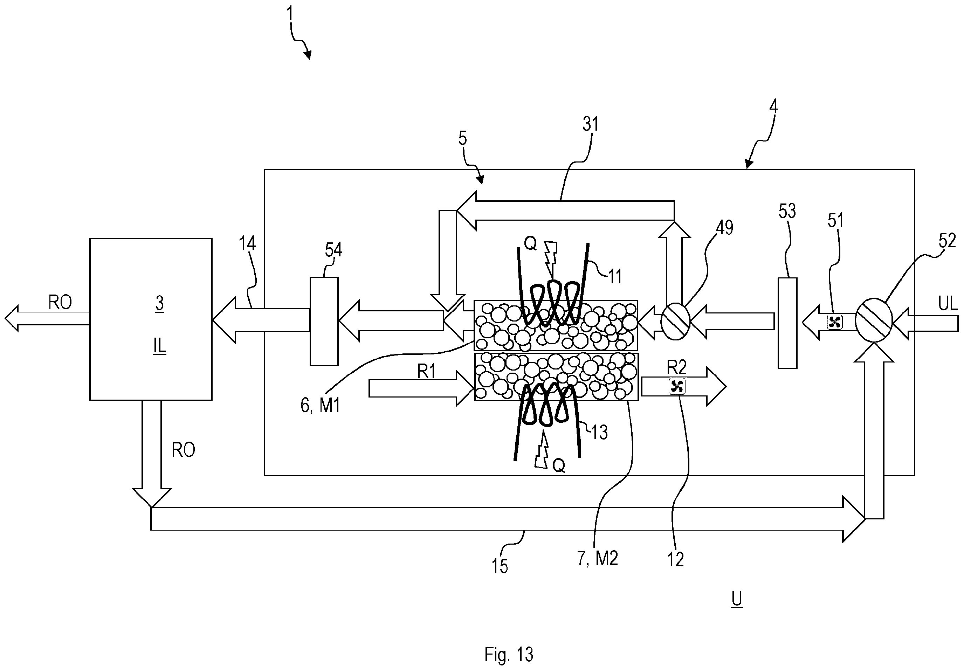

Auch die Fig. 13 zeigt eine Weiterbildung des Kraftfahrzeugs 1, bei der die Vorrichtung 5 in die Klimaanlage 4 vollintegriert ist. Auch bei dieser Ausführungsform der Vorrichtung 5 ist die erste Gebläseeinrichtung 10 verzichtbar. Hierbei sind auch die Umgehungsleitung 31 und das Umgehungsventil 49 in die Klimaanlage 4 integriert. Es ist somit möglich, von der Umgebung U angesaugte Umgebungsluft UL mit Hilfe der Gebläseeinrichtung 51 anzusaugen und je nach Schaltzustand des Umgehungsventils 49 vollständig um die Sorptionseinheiten 6, 7 herumzuleiten oder vollständig oder teilweise durch eine der Sorptionseinheiten 6, 7 hindurchzuleiten.

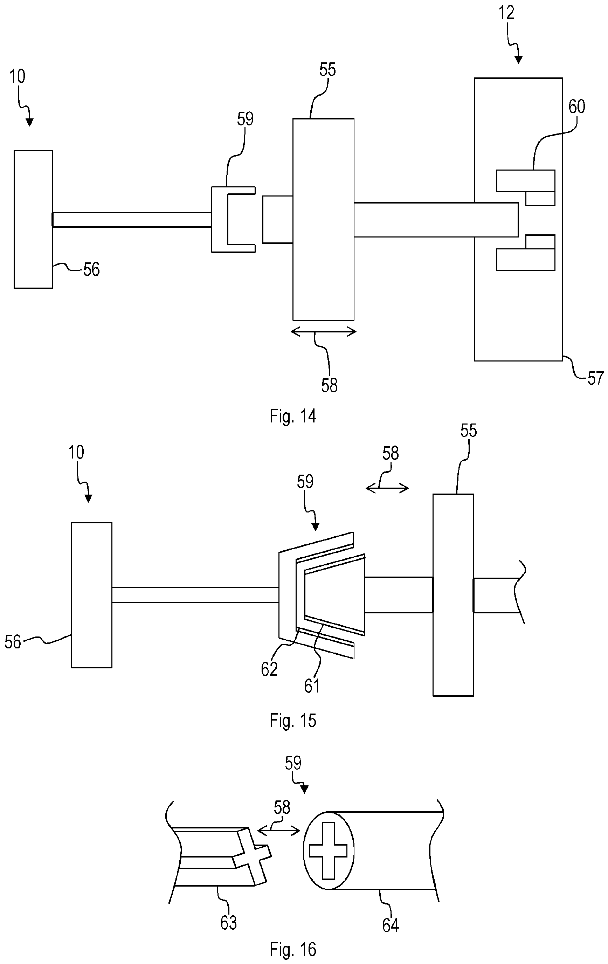

Wie die Fig. 14 zeigt, können die beiden Gebläseeinrichtungen 10, 12 der Vorrichtung 5 von einem gemeinsamen Antriebselement 55, beispielsweise von einem Elektromotor, angetrieben werden. Dabei ist der ersten Gebläseeinrichtung 10 ein erstes Gebläserad 56 und der zweiten Gebläseeinrichtung 12 ist ein zweites Gebläserad 57 zugeordnet. Das Antriebselement 55 kann, wie mit Hilfe eines Pfeils 58 gezeigt, linear verschieblich sein, um das Antriebselement 55 wahlweise mit den Gebläserädern 56, 57 zu koppeln und von diesen zu entkoppeln.

Das Antriebselement 55 weist dabei drei Schaltpositionen auf. In einer ersten Schaltposition wird nur das zweite Gebläserad 57 angetrieben, in einer zweiten Schaltposition werden beide Gebläserräder 56, 57 angetrieben und in einer dritten Schaltposition wird nur das erste Gebläserad 56 angetrieben. Zwischen dem ersten Gebläserad 56 und dem Antriebselement 55 kann eine erste Kupplung 59 vorgesehen sein, die geeignet ist, das Antriebselement 55 mit dem ersten Gebläserad 56 zu koppeln oder dieses von dem Antriebselement 55 zu entkoppeln. Zwischen dem zweiten Gebläserad 57 und dem Antriebselement 55 kann eine zweite Kupplung 60 vorgesehen sein, die geeignet ist, das Antriebselement 55 mit dem zweiten Gebläserad 57 zu koppeln oder dieses von dem Antriebselement 55 zu entkoppeln.

Wie die Fig. 15 zeigt, kann die erste Kupplung 59 als kegelförmiges Zahnradgetriebe mit zwei kegelförmigen Zahnrädern 61, 62 ausgebildet sein. Dabei ist dem Antriebselement 55 das Zahnrad 61 und dem ersten Gebläserad 56 das Zahnrad 62 zugeordnet. Durch ein axiales Verschieben des Antriebselements 55 können die Zahnräder 61, 62 in formschlüssigen Eingriff und wieder außer Eingriff gebracht werden. Die zweite Kupplung 60 kann identisch wie die erste Kupplung 59 aufgebaut sein.

Wie die Fig. 16 zeigt, können die Kupplungen 59, 60 auch als kreuzförmige Zahnradgetriebe ausgebildet sein. In diesem Fall kann die jeweilige Kupplung 59, 60 jeweils zwei Kreuzwellen 63, 64 umfassen, die ineinander steckbar und auseinanderziehbar sind, um das Antriebselement 55 an das jeweilige Gebläserad 56, 57 zu koppeln oder das jeweilige Gebläserad 56, 57 von dem Antriebselement 55 zu entkoppeln.

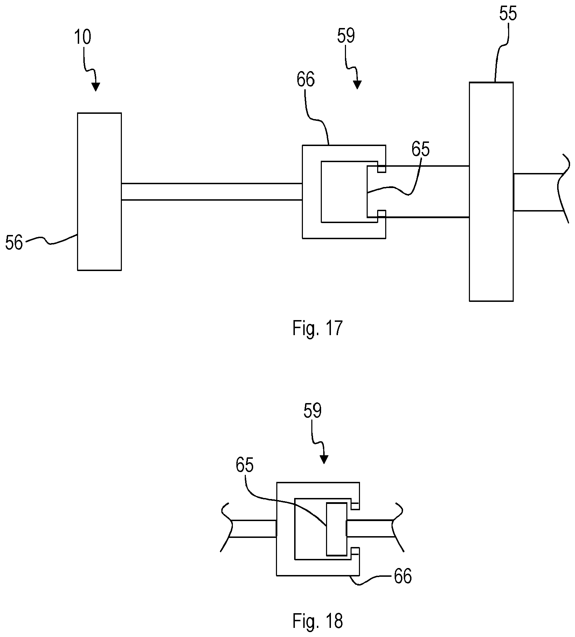

Wie die Fig. 17 und 18 zeigen, kann auch jeweils eine aktuierte Kupplung 59, 60 mit einem ersten Kupplungselement 65 und einem zweiten Kupplungselement 66 vorgesehen sein. Die Kupplungselemente 65, 66 sind mit Hilfe eines nicht gezeigten Aktuators aktuierbar um die Kupplungselemente 65, 66 miteinander zu verbinden oder voneinander zu trennen. Mit Hilfe des Verwendens der Kupplungen 59, 60 kann somit ein Antriebselement 55 eingespart werden.

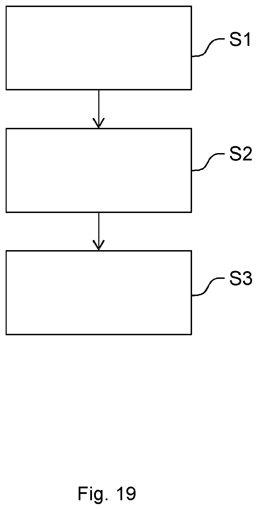

Die Fig. 19 zeigt ein schematisches Blockdiagramm eines Verfahrens zum Betreiben der zuvor erläuterten Vorrichtung 5. Das Verfahren umfasst die nachfolgend erläuterten Schritte. In einem Schritt S1 wird eine der beiden Sorptionseinheiten 6, 7, beispielsweise die erste Sorptionseinheit 6, in Abhängigkeit von dem Kohlendioxid- und Wassergehalt in dem Fahrgastraum 3 mit Hilfe der Luftverteilungseinrichtung 19 in den Sorptionsmodus M1 geschaltet. In dem Sorptionsmodus M1 werden, wie zuvor erwähnt, von der entsprechenden Sorptionseinheit 6, 7 Kohlendioxid und Wasser aus der Rohluft RO des Fahrgastraums 3 sorbiert.

In einem Schritt S2 wird die andere der beiden Sorptionseinheiten 6, 7, beispielsweise die zweite Sorptionseinheit 7 mit Hilfe der Luftverteilungseinrichtung 19 in den Desorptionsmodus M2 geschaltet, in dem von der entsprechenden Sorptionseinheit 6, 7 Kohlendioxid und Wasser an die zugeführte unbeladene Regenerationsluft R1 desorbiert und an die Umgebung U abgegeben.

In einem Schritt S3 werden die Schritte S1 und S2 derart wechselweise durchgeführt, dass immer eine der beiden Sorptionseinheiten 6, 7 in dem Sorptionsmodus M1 betrieben wird, während die andere der beiden Sorptionseinheiten 6, 7 in dem Desorptionsmodus M2 betrieben wird.

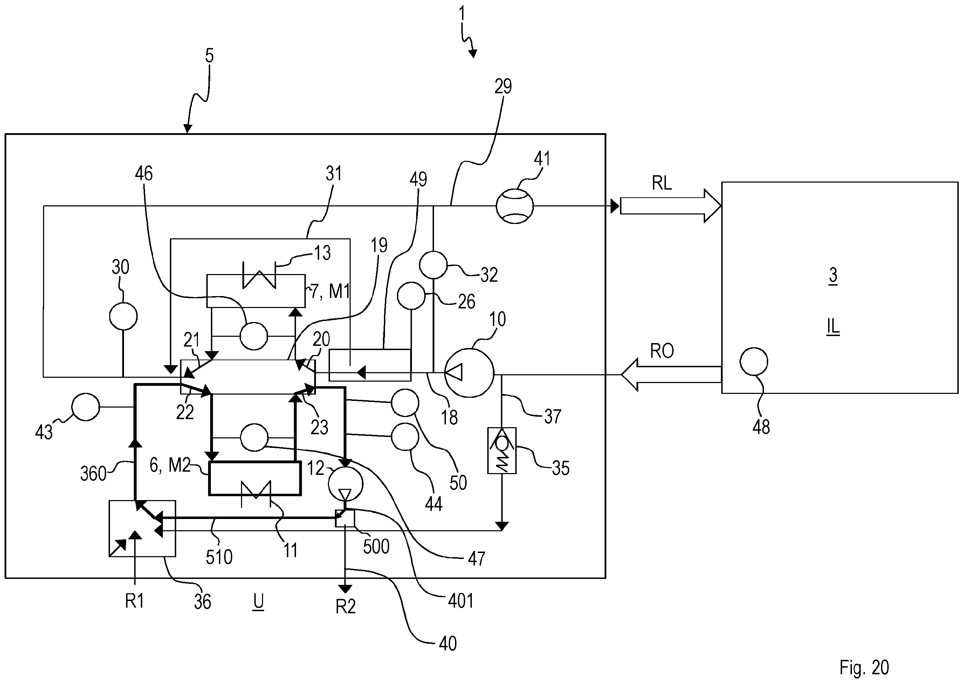

In den hydraulischen Schaltbildern des Kraftfahrzeugs 1 mit Vorrichtung 5 zum kombinierten Reduzieren des Kohlendioxid- und Wassergehalts in einem umgrenzten Luftvolumen der **Fig. 20****,** **Fig. 21** und **Fig. 22****,** welche jeweils Weiterbildungen der Fig. 5 darstellen, ist wiederum die Desorptionsgebläseeinrichtung 12 aktiv. Die **fett** dargestellten Fluidpfade sind dabei die bei den gewählten Schaltstellungen aktiven.

Gemäß **Fig. 20** befindet sich die Sorptionseinheit 6 im Desorptionsmodus M2 und wird von einem Regenerationsluftvolumenstrom R1 durchströmt. Der Desorptionsgebläseeinrichtung 12 druckseitig nachgeschaltet ist Abzweig 401 in der Desorptionsleitung 40 vorgesehen, der mittels eines Rezirkulationsventils 500 schaltbar ist. Der Abzweig 401 mündet in eine Rezirkulationsleitung 510, die fluidisch mit einem Eingang des Regenerationsventils 36 verbunden ist, wobei mittels des Regenerationsventils 36 eine vierte Schaltstellung, in welcher der sich im Desorptionsmodus befindlichen Sorptionseinheit 6 die Regenerationsluft R1 aus der Rezirkulationsleitung 510 zuführbar ist, schaltbar ist und umgekehrt, sodass mittels der Desorptionsgebläseeinrichtung 12 die Regerationsluft R1 wahlweise durch die Sorptionseinheiten 6,7 rezirkulierbar oder in die Umgebung leitbar ist.

Dies hat Vorteile hinsichtlich Energieverbrauch, da die zur Regeneration einmal eingebrachte Wärmemenge optimal zum Desorbieren eingesetzt wird und Wärmeverluste dadurch verringert werden.

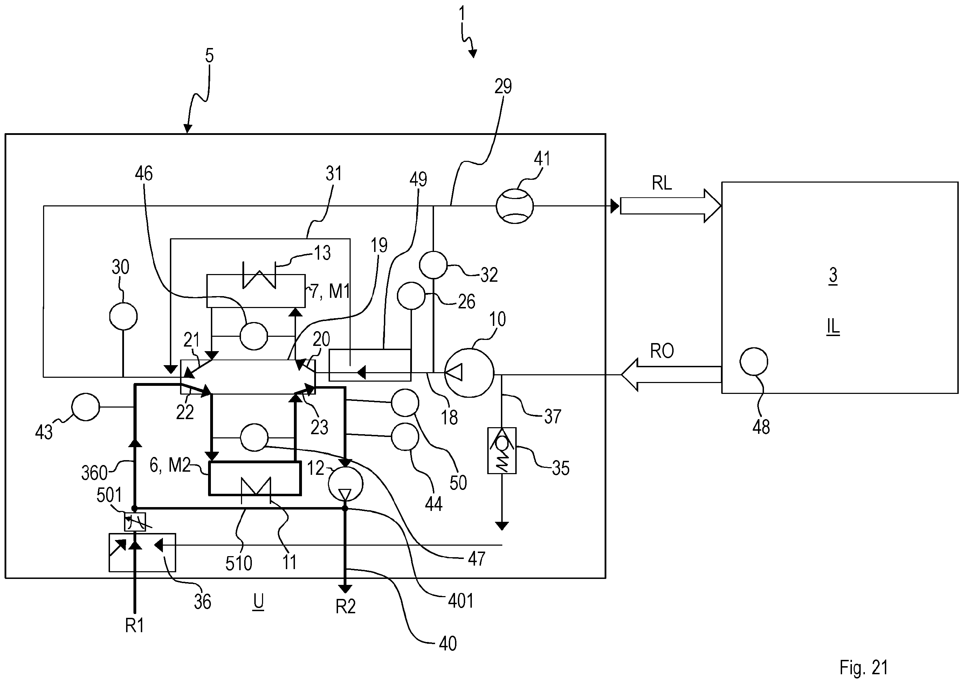

Eine alternative Ausführungsform, gemäß der ebenfalls eine zumindest teilweise Rezirkulation der zumindest teilweise beladenen Rezirkulationsluft R2, d. h. des Luftvolumenstroms, welcher die Sorptionseinheit 6, welche sich im Desorptionsmodus befindet, zumindest einmal passiert hat, möglich ist, ist in der **Fig. 21** dargestellt.

Demgemäß ist fluidisch zwischen dem Abgang des Regenerationsventils und der Einmündung der Rezirkulationsleitung in die Regenerationsluftleitung ein Drosselventil 501, insbesondere ein einstellbares Drosselventil 501 angeordnet, mittels welchem der Anteil an frisch zugeführter unbeladener Regenerationsluft R1 eingestellt werden kann. Gemäß dieser Ausführungsform ist an dem Abzweig 401 kein Rezirkulationsventil 500 vorgesehen, sondern der Abzweig ist als ungeschalteter Abzweig (z. B. Verteiler, T-Stück, Y-Stück o. ä.) ausgebildet. Dies hat den Vorteil, dass die Desorption der Sorptionseinheit, welche sich in dem Desorptionsmodus befindet, stets unter einem hinreichenden Konzentrationsgefälle erfolgen kann, was hilft, die Gesamtregenerationsdauer zu minimieren.

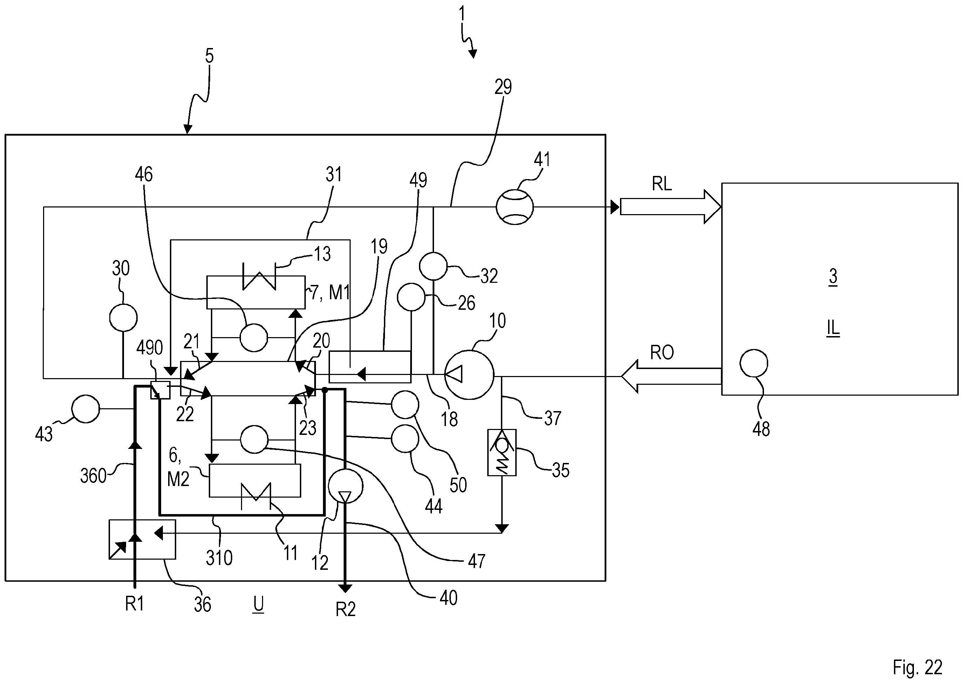

In **Fig. 22** ist eine Weiterbildung gezeigt, die eine zweite Umgehungsleitung 310 sowie ein zweites Umgehungsventil 490 aufweist. Das zweite Umgehungsventil 490 weist zwei Schaltzustände auf, zwischen denen es wahlweise schaltbar ist. Gemäß dem ersten Schaltzustand ist Regenerationsluft R1 derjenigen Sorptionseinheit 6, 7, welche sich in dem Desorptionsmodus M2 befindet, zuführbar. Gemäß dem zweiten Schaltzustand ist Regenerationsluft R1 mit Hilfe der Umgehungsleitung 310 unter Umgehung der Sorptionseinheiten 6, 7 in die Umgebung U leitbar. Hierbei handelt es sich in erster Linie um einen Test- bzw. Kalibrierungsmodus, eingesetzt werden kann, um ggf. im System vorgesehene Sensoren zu prüfen. Oder im Sinne eines "Notlaufmodus" wenn der Druckverlust über eine der Sorptionseinheiten einen vorbestimmten Grenzwert übersteigt.

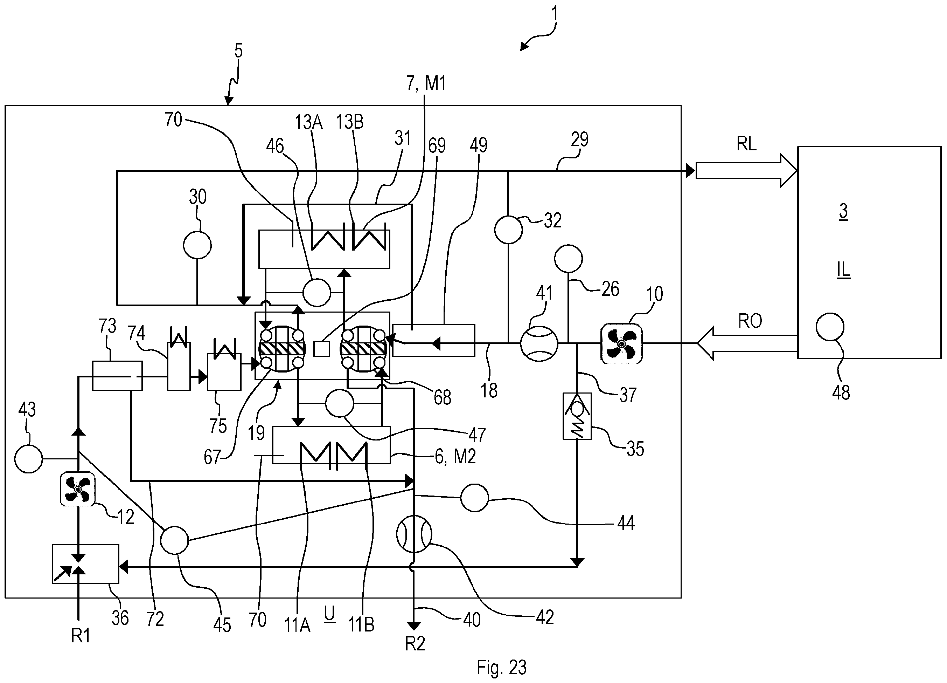

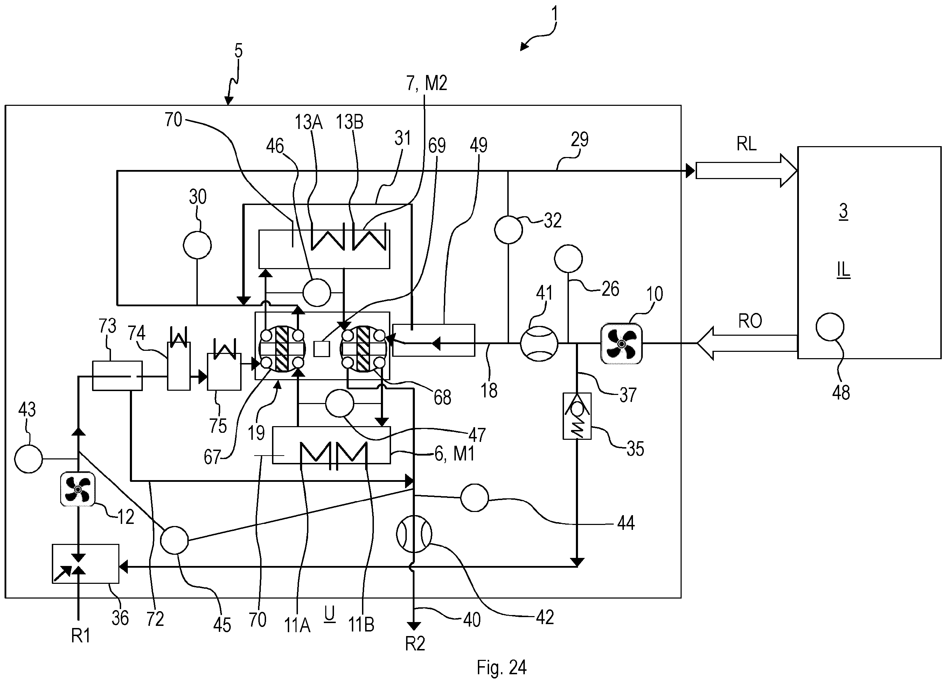

Die Fig. 23 und 24 zeigen das Kraftfahrzeug 1 mit einer Weiterbildung der Vorrichtung 5. Bei dieser Ausführungsform des Kraftfahrzeugs 1 arbeitet die Vorrichtung 5 ebenfalls unabhängig von der Klimaanlage 4. Im Betrieb des Kraftfahrzeugs 1 belädt sich die Innenraumluft IL in dem Fahrgastraum 3 mit CO2 und H2O. Die beladene Innenraumluft IL ist die zuvor erwähnte Rohluft RO. Die mit CO2 und H2O beladene Rohluft RO kann zumindest teilweise der Vorrichtung 5 und somit den Sorptionseinheiten 6, 7 zugeführt werden. Insbesondere wird die Rohluft RO einer ersten Gebläseeinrichtung 10 zugeführt. Die erste Gebläseeinrichtung 10 ist dabei druckseitig montiert. Die erste Gebläseeinrichtung 10 kann jedoch auch saugseitig montiert werden.

Stromabwärts der ersten Gebläseeinrichtung 10 ist eine Leitung 18 vorgesehen, welche die Rohluft RO einer Luftverteilungseinrichtung 19 zuführt. Die Luftverteilungseinrichtung 19 umfasst mehrere Klappeneinheiten 67, 68. Dementsprechend kann die Luftverteilungseinrichtung 19 auch als Klappeneinrichtung oder Klappensystem bezeichnet werden. Die Klappenheinheiten 67, 68 können von einem gemeinsamen Klappenaktuator 69 angesteuert werden. Eine erste Klappeneinheit 67 kann wahlweise mit der ersten Sorptionseinheit 6 oder mit der zweiten Sorptionseinheit 7 in Fluidverbindung gebracht werden. Dementsprechend kann eine zweite Klappeneinheit 68 ebenfalls wahlweise mit der ersten Sorptionseinheit 6 oder mit der zweiten Sorptionseinheit 7 in Fluidverbindung gebracht werden.

Die Leitung 18 ist mit der Luftverteilungseinrichtung 19 in Fluidverbindung, die wiederum mit den beiden Sorptionseinheiten 6, 7 in Fluidverbindung ist. Je nach Schaltstellung der Klappeneinheiten 67, 68 kann die Rohluft RO wahlweise entweder der ersten Sorptionseinheit 6 oder der zweiten Sorptionseinheit 7 zugeführt werden. In oder an der Leitung 18 kann ein Sensor 26, insbesondere ein CO2-, H20- und/oder Temperatursensor, vorgesehen sein.

Eine Leitung 29 verbindet die Luftverteilungseinrichtung 19 mit dem Fahrgastraum 3. Auch die Leitung 29 kann einen Sensor 30, insbesondere einen CO2-, H20- und/oder Temperatursensor, umfassen. Mit Hilfe des Sensors 30 kann beispielsweise erfasst werden, wenn die sich im Sorptionsmodus M1 befindliche Sorptionseinheit 6, 7 erschöpft ist. Zwischen den Leitungen 18, 29 ist eine Umgehungsleitung 31, insbesondere eine Adsorberbypassleitung, zum Umgehen der Sorptionseinheiten 6, 7 vorgesehen. Ferner ist zwischen den Leitungen 18, 29 auch ein Sensor 32, insbesondere ein Drucksensor, vorgesehen, der eine Druckdifferenz zwischen den Leitungen 18, 29 erfassen kann.

Die Vorrichtung 5 umfasst ein Regenerationsventil 36. Das Regenerationsventil 36 ist vorzugsweise ein Dreiwegeventil. Dem Regenerationsventil 36 wird die Umgebungsluft UL als unbeladene Regenerationsluft R1 zugeführt. Des Weiteren kann dem Regenerationsventil 36 auch über eine Leitung 37 die Innenraumluft IL als unbeladene Regenerationsluft R1 zugeführt werden. In oder an der Leitung 37 ist ein Rückschlagventil 35 vorgesehen.

Über eine Leitung 40 kann beladene Regenerationsluft R2 an die Umgebung U abgegeben werden. Die Leitung 40 umfasst einen Volumenstromsensor 42. Der Volumenstromsensor 42 kann als Desorptionsvolumenstromsensor bezeichnet werden. Stromabwärts des Regenerationsventils 36 ist eine zweite Gebläseeinrichtung 12 vorgesehen. Die zweite Gebläseeinrichtung 12 ist dabei druckseitig, das heißt stromaufwärts der Sorptionseinheiten 6, 7 positioniert. Die zweite Gebläseeinrichtung 12 kann jedoch auch saugseitig, das heißt stromabwärts der Sorptionseinheiten 6, 7 positioniert sein. Die zweite Gebläseeinrichtung 12 gibt die beladene Regeneratiosluft R2 an die Umgebung U ab.

Der ersten Gebläseeinrichtung 10 kann ein Volumenstromsensor 41 nachgeschaltet sein. Stromabwärts der Luftverteilungseinrichtung 19 ist in der Leitung 40 ein Volumenstromsensor 42 vorgesehen. Stromabwärts der zweiten Gebläseeinrichtung 12 ist ein weiterer Sensor 43, insbesondere ein CO2-, H20- und/oder Temperatursensor, vorgesehen. Ferner ist auch in oder an der Leitung 40 ein weiterer Sensor 44, insbesondere ein CO2-, H20- und/oder Temperatursensor, vorgesehen. Ferner ist ein Sensor 45 vorgesehen, der geeignet ist, eine Druckdifferenz zwischen der Leitung 40 und der Luftverteilungseinrichtung 19 zu erfassen. Der Sensor 45 ist stromabwärts des Sensors 43 vorgesehen.

Jeder Sorptionseinheit 6, 7 ist ein Sensor 46, 47, insbesondere ein Differenzdrucksensor, zugeordnet. Auch in dem Fahrgastraum 3 kann ein Sensor 48, insbesondere ein CO2-, H20- und/oder Temperatursensor, vorgesehen sein. Um die Umgehungsleitung 31 zu aktivieren und zu deaktivieren, ist ein Umgehungsventil 49 vorgesehen. Jede Sorptionseinheit 6, 7 umfasst mehrere Temperatursensoren 70, 71. Beispielsweise sind jeder Sorptionseinheit 6, 7 vier derartige Temperatursensoren 70, 71 zugeordnet. Jede Sorptionseinheit 6, 7 kann mehrere Heizelemente 11A, 11B, 13A, 13B umfassen. Die Heizelemente 11A, 11B, 13A, 13B sind optional. Die Heizelemente 11A, 11B beziehungsweise 13A, 13B können sandwichartig angeordnet sein. Die Vorrichtung 5 umfasst eine weitere Leitung 72, insbesondere eine Regenerationsbypassleitung. Die Leitung 72 mündet stromaufwärts des Volumenstromsensors 42 in die Leitung 40 ein. Mit Hilfe der Leitung 72 kann die Luftverteilungseinrichtung 19 umgangen werden. Die Leitung 72 führt von einem Umgehungsventil 73, das stromabwärts der Luftverteilungseinrichtung 19 angeordnet ist, hin zu der Leitung 40.

Stromabwärts des Umgehungsventils 73, das heißt zwischen dem Umgehungsventil 73 und der Luftverteilungseinrichtung 19 ist ein optionales Heizelement 74, insbesondere ein Wärmetauscher, vorgesehen. Mit Hilfe des Heizelements 74 kann beispielsweise Abwärme eines Elektromotors, eines Verbrennungsmotors oder einer Batteriekühlung auf die unbeladene Regenerationsluft R1 übertragen werden. Stromabwärts des Heizelements 74 ist ein optionales weiteres Heizelement 75 vorgesehen. Das Heizelement 75 ist zwischen dem Heizelement 74 und der Luftverteilungseinrichtung 19 angeordnet. Das Heizelement 75 ist ebenfalls geeignet, Wärme auf die unbeladene Regenerationsluft R1 zu übertragen. Das Heizelement 75 kann beispielsweise ein elektrisches Heizelement sein.

Die Fig. 23 zeigt eine Schaltstellung der Luftverteilungseinrichtung 19, in welcher sich die erste Sorptionseinheit 6 in dem Desorptionsmodus M2 und in welcher sich die zweite Sorptionseinheit 7 in dem Sorptionsmodus M1 befindet. Die zweite Klappeneinheit 68 ist derart geschaltet, dass die Rohluft RO mit Hilfe der ersten Gebläseeinrichtung 10 der zweiten Sorptionseinheit 7 zugeführt wird. Die erste Klappeneinheit 67 ist derart geschaltet, dass die von H2O und CO2 gereinigte Rohluft RO dem Fahrgastraum 3 über die Leitung 29 nun als Reinluft RL zugeführt wird.

Wie zuvor erwähnt, befindet sich die erste Sorptionseinheit 6 in dem Desorptionsmodus M2. Hierzu wird über das Regenerationsventil 36 und die zweite Gebläseeinrichtung 12 unbeladene Regenerationsluft R1 aus der Umgebung U angesaugt. Die unbeladene Regenerationsluft R1 wird dem bevorzugt als Wärmetauscher ausgebildeten Heizelement 74 zugeführt und vorgewärmt. Zusätzlich kann die Regenerationsluft R1 mit Hilfe des dem Heizelement 74 nachgeschalteten optionalen Heizelements 75 weiter erwärmt werden. Über die erste Klappeneinheit 67 der Luftverteilungseinrichtung 19 wird die erwärmte Regenerationsluft R1 der ersten Sorptionseinheit 6 zugeführt. Optional kann die erste Sorptionseinheit 6 selbst auch Heizelemente 11A, 11B aufweisen, die in dem Desorptionsmodus M2 aktiviert werden. Dies ist jedoch nicht zwingend erforderlich. Die zweite Klappeneinheit 68 ist derart geschaltet, dass die beladene Regenerationsluft R2 über die Leitung 40 in die Umgebung U abgeführt wird.

Die Fig. 24 zeigt eine Schaltstellung der Luftverteilungseinrichtung 19, in welcher sich die erste Sorptionseinheit 6 in dem Sorptionsmodus M1 und in welcher sich die zweite Sorptionseinheit 7 in dem Desorptionsmodus M2 befindet. Hierzu werden die Klappeneinheiten 67, 68 der Luftverteilungseinrichtung 19 entsprechend geschaltet. Das Heizelement 74 und das Heizelement 75 werden genutzt, um die der zweiten Sorptionseinheit 7 zugeführte unbeladene Regenerationsluft R1 zu erwärmen. Das Heizelement 74 und das Heizelement 75 können somit für den Desorptionsmodus M2 beider Sorptionseinheiten 6, 7 eingesetzt werden.

Dadurch, dass das Heizelement 74 und/oder das Heizelement 75 nicht in die Sorptionseinheiten 6, 7 integriert sind, sondern in Strömungsrichtung diesen vorgelagert sind, nämlich vor der Einströmung in die Luftverteilungseinrichtung 19, kann der Wärmeeintrag in die Sorptionseinheiten 6, 7 durch den Luftstrom homogener erfolgen als bei einer in-situ Erwärmung über die elektrischen Heizelemente 11A, 11B, 13A, 13B in den Sorptionseinheiten 6, 7. Bei einer Erwärmung über den Luftstrom erfolgt die Wärmeübertragung hauptsächlich durch Konvektion, wohingegen bei einer Erwärmung mit Hilfe der Heizelemente ein hoher Wärmeleitungsanteil vorliegt. Durch die Verwendung der externen Heizelemente 74, 75 erfolgt die Desorption schneller. Ferner lässt sich der Abschluss des Desorptionsmodus M2 einfacher messtechnisch erfassen, wobei das Endkriterium für den Abschluss der Desorption ist, dass die Eingangstemperatur gleich der Ausgangstemperatur ist. Ein weiterer Vorteil ist, dass nur ein Heizelement 74 beziehungsweise nur ein Heizelement 75 benötigt wird, das beide Sorptionseinheiten 6, 7 beheizen kann.

### Verwendete Bezugszeichen:

- 1: Kraftfahrzeug
- 2: Karosserie
- 3: umgrenztes Luftvolumen/Fahrgastraum
- 4: Klimaanlage
- 5: Vorrichtung
- 6: Sorptionseinheit
- 7: Sorptionseinheit
- 8: Sorbens
- 9: Sorbens
- 10: Gebläseeinrichtung
- 11: Heizelement
- 11A: Heizelement
- 11B: Heizelement
- 12: Gebläseeinrichtung
- 13: Heizelement
- 13A: Heizelement
- 13B: Heizelement
- 14: Leitung
- 15: Leitung
- 16: Leitung
- 17: Leitung
- 18: Leitung
- 19: Luftverteilungseinrichtung
- 20: Ventil
- 21: Ventil
- 22: Ventil
- 23: Ventil
- 24: Leitung
- 25: Leitung
- 26: Sensor
- 27: Leitung
- 28: Leitung
- 29: Leitung
- 30: Sensor
- 31: Umgehungsleitung
- 310: Zweite Umgehungsleitung
- 32: Sensor
- 33: Leitung
- 34: Leitung
- 35: Rückschlagventil
- 36: Regenerationsventil
- 360: Regenerationsluftleitung
- 37: Leitung
- 38: Leitung
- 39: Leitung
- 40: (Desorptions-)Leitung
- 401: Abzweig der Desorptionsleitung
- 41: Volumenstromsensor
- 42: Volumenstromsensor
- 43: Sensor
- 44: Sensor
- 45: Sensor
- 46: Sensor
- 47: Sensor
- 48: Sensor
- 49: Umgehungsventil
- 490: Zweites Umgehungsventil
- 50: Drucksensor
- 500: Rezirkulationsventil
- 501: Drosselventil, einstellbares
- 510: Rezirkulationsleitung
- 51: Gebläseeinrichtung
- 52: Ventil
- 53: Kühlelement
- 54: Heizelement
- 55: Antriebselement
- 56: Gebläserad
- 57: Gebläserad
- 58: Pfeil
- 59: Kupplung
- 60: Kupplung
- 61: Zahnrad
- 62: Zahnrad
- 63: Kreuzwelle
- 64: Kreuzwelle
- 65: Kupplungselement
- 66: Kupplungselement
- 67: Klappeneinheit
- 68: Klappeneinheit
- 69: Klappenaktuator
- 70: Temperatursensor
- 71: Temperatursensor
- 72: Leitung
- 73: Umgehungsventil
- 74: Heizelement
- 75: Heizelement

- IL: Innenraumluft
- M1: Sorptionsmodus
- M2: Desorptionsmodus
- RL: Reinluft
- RO: Rohluft
- R1: unbeladene Regenerationsluft
- R2: beladene Regenerationsluft
- S1: Schritt
- S2: Schritt
- S3: Schritt
- U: Umgebung
- UL: Umgebungsluft
- Q: Wärme

## Patentansprüche

1. Vorrichtung (5) zum kombinierten Reduzieren des Kohlendioxid- und Wassergehalts in einem umgrenzten Luftvolumen (3), nämlich in einem Fahrgastraum eines Kraftfahrzeugs (1), mit einer ersten Sorptionseinheit (6) zum kombinierten Sorbieren von Kohlendioxid und Wasser, einer zweiten Sorptionseinheit (7) zum kombinierten Sorbieren von Kohlendioxid und Wasser, wobei die erste Sorptionseinheit (6) und die zweite Sorptionseinheit (7) jeweils mehrere Sorbenzien (8, 9) enthalten, wobei die Sorptionseinheiten (6, 7) jeweils von einem Sorptionsmodus (M1), in dem die Sorptionseinheiten (6, 7) Kohlendioxid und Wasser aus Rohluft (RO) des umgrenzten Luftvolumens (3) sorbieren, in einen Desorptionsmodus (M2), in dem die Sorptionseinheiten (6, 7) Kohlendioxid und Wasser an zugeführte Regenerationsluft (R1) desorbieren, und umgekehrt verbringbar sind, und einer Luftverteilungseinrichtung (19), mit deren Hilfe die Sorptionseinheiten (6, 7) in Abhängigkeit von dem Kohlendioxid- und Wassergehalt in dem umgrenzten Luftvolumen (3) jeweils von dem Sorptionsmodus (M1) in den Desorptionsmodus (M2) und umgekehrt derart wechselweise schaltbar sind, dass sich in zumindest einem Betriebszustand der Vorrichtung (5) eine der beiden Sorptionseinheiten (6, 7) in dem Sorptionsmodus (M1) befindet, während sich die andere der beiden Sorptionseinheiten (6, 7) in dem Desorptionsmodus (M2) befindet, wobei in dem Desorptionsmodus (M2) die durch die Sorptionseinheit (6, 7), welche sich in dem Desorptionsmodus befindet, geleitete Regenerationsluft (R1) als beladene Regenerationsluft (R2) einer Umgebung (U) zuführbar ist, insbesondere über eine Desorptionsleitung (40), und wobei die Regenerationsluft (R1) dem umgrenzten Luftvolumen (3) oder der Umgebung (U) entnehmbar ist, ferner umfassend ein Heizelement (11, 11A, 11B, 13, 13A, 13B, 74, 75) zum Einbringen von Wärme (Q) in diejenige Sorptionseinheit (6, 7), die sich in dem Desorptionsmodus (M2) befindet, wobei das Heizelement (74, 75) stromaufwärts der Sorptionseinheiten (6, 7) positioniert ist und Wärme in die unbeladene Regenerationsluft (R1) einbringt.

2. Vorrichtung nach Anspruch 1, wobei die Luftverteilungseinrichtung (19) mehrere Ventile (20 - 23) aufweist, die derart schaltbar sind, dass im Betrieb der Vorrichtung (5) derjenigen Sorptionseinheit (6, 7), die sich in dem Sorptionsmodus (M1) befindet, die Rohluft (RO) aus dem umgrenzten Luftvolumen (3) zuführbar ist, um das Kohlendioxid und das Wasser aus der Rohluft (RO) zu entfernen, und derjenigen Sorptionseinheit (6, 7), die sich in dem Desorptionsmodus (M2) befindet, die Regenerationsluft (R1) zuführbar ist, um das Kohlendioxid und das Wasser aus der Sorptionseinheit (6, 7) zu entfernen.

3. Vorrichtung nach Anspruch 1 oder 2, wobei in jede Sorptionseinheit (6, 7) ein Heizelement (11, 11A, 11B, 13, 13A, 13B) integriert ist.

4. Vorrichtung nach Anspruch 1 oder 2, wobei die Sorptionseinheiten (6, 7) ein gemeinsames Heizelement (74, 75) aufweisen.

5. Vorrichtung nach einem der Ansprüche 1 - 4, ferner umfassend ein Regenerationsventil (36), welches eine erste Schaltstellung, in welcher der sich im Desorptionsmodus (M2) befindlichen Sorptionseinheit (6, 7) die Regenerationsluft (R1) aus einer Umgebung (U) des umgrenzten Luftvolumens (3) zuführbar ist, eine zweite Schaltstellung, in welcher der sich im Desorptionsmodus (M2) befindlichen Sorptionseinheit (6, 7) die Regenerationsluft (R1) aus dem umgrenzten Luftvolumen (3) zuführbar ist, und insbesondere eine dritte Schaltstellung aufweist, in welcher die sich im Desorptionsmodus (M2) befindliche Sorptionseinheit (6, 7) unter Vakuum regenerierbar ist.

6. Vorrichtung nach einem der Ansprüche 1 - 5, ferner umfassend eine Gebläseeinrichtung (10), die derjenigen Sorptionseinheit (6, 7), die sich im Sorptionsmodus (M1) befindet, die Rohluft (RO) zuführt.

7. Vorrichtung nach Anspruch 6, wobei die Gebläseeinrichtung (10, 51) Teil einer Klimaanlage (4) ist.

8. Vorrichtung nach einem der Ansprüche 1 - 7, ferner umfassend eine Desorptionsgebläseeinrichtung (12), die derjenigen Sorptionseinheit (6, 7), die sich in dem Desorptionsmodus (M2) befindet, die Regenerationsluft (R1) zuführt, wobei die Desorptionsgebläseeinrichtung (12) bezüglich der Sorptionseinheiten (6, 7) druckseitig oder saugseitig angeordnet ist.

9. Vorrichtung nach einem der Ansprüche 1 - 8, ferner umfassend zumindest eine Umgehungsleitung (31) und zumindest ein Umgehungsventil (49), das wahlweise von einem ersten Schaltzustand, in welchem die Rohluft (RO) derjenigen Sorptionseinheit (6, 7), welche sich in dem Sorptionsmodus (M1) befindet, zuführbar ist, in einen zweiten Schaltzustand, in welchem die Rohluft (RO) mit Hilfe der Umgehungsleitung (31) um die Sorptionseinheiten (6, 7) herum zurück in das umgrenzte Luftvolumen (3) leitbar ist, und umgekehrt schaltbar ist.

10. Vorrichtung nach einem der Ansprüche 1 - 9, ferner umfassend eine zweite Umgehungsleitung (310) sowie ein zweites Umgehungsventil (490), das wahlweise von einem ersten Schaltzustand, in welchem die Regenerationsluft (R1) derjenigen Sorptionseinheit (6, 7), welche sich in dem Desorptionsmodus (M2) befindet, zuführbar ist, in einen zweiten Schaltzustand, in welchem die Regenerationsluft (R1) mit Hilfe der Umgehungsleitung (310) unter Umgehung der Sorptionseinheiten (6, 7) in die Umgebung (U) führbar ist, und umgekehrt schaltbar ist.

11. Vorrichtung nach Anspruch 8, wobei der Desorptionsgebläseeinrichtung (12) druckseitig nachgeschaltet ein Abzweig (401) in der Desorptionsleitung (40) vorgesehen ist, der in eine Rezirkulationsleitung (510) mündet, die fluidisch mit einem Eingang eines Regenerationsventils (36) verbunden ist oder stromabwärts eines Abgangs des Regenerationsventils (36) in eine an den Abgang angeschlossene Regenerationsluftleitung (360) einmündet.

12. Vorrichtung nach Anspruch 11, wobei der Abzweig (401) mittels eines Rezirkulationsventils (500) schaltbar ist, sodass mittels der Desorptionsgebläseeinrichtung (12) die Regerationsluft (R1) wahlweise durch die Sorptionseinheiten (6, 7) rezirkulierbar oder in die Umgebung (U) leitbar ist.

13. Vorrichtung nach Anspruch 11 oder 12, wobei die Rezirkulationsleitung (510) fluidisch mit dem Regenerationsventil (36) verbunden ist, wobei mittels des Regenerationsventils (36) eine vierte Schaltstellung, in welcher der sich im Desorptionsmodus (M2) befindlichen Sorptionseinheit (6, 7) die Regenerationsluft (R1) aus der Rezirkulationsleitung (510) zuführbar ist, und umgekehrt schaltbar ist.

14. Vorrichtung nach Anspruch 11 oder 12, wobei fluidisch zwischen dem Abgang des Regenerationsventils (36) und der Einmündung der Rezirkulationsleitung (510) in die Regenerationsluftleitung (360) ein Drosselventil, insbesondere ein einstellbares Drosselventil (501), angeordnet ist.

15. Vorrichtung nach einem der Ansprüche 1 - 14, wobei die Sorptionseinheiten (6, 7) dazu geeignet sind, die Rohluft (RO) neben Kohlendioxid und Wasser auch von Feinpartikeln, Stickoxiden und/oder flüchtigen organischen Verbindungen zu befreien.

16. Vorrichtung nach Anspruch 15, wobei die Sorptionseinheiten (6, 7) ein erstes Sorbens (8), das geeignet ist, Kohlendioxid zu adsorbieren, ein zweites Sorbens (9), das geeignet ist, Wasser zu adsorbieren, und weitere Sorbenzien, die geeignet sind, die Rohluft (RO) von Feinpartikeln, Stickoxiden und/oder flüchtigen organischen Verbindungen zu befreien, aufweisen, wobei die weiteren Sorbenzien bevorzugt zwischen zwei Trägerlagen, insbesondere aus einem Vlies, eingebracht sind, oder wobei die Sorbenzien (8, 9) und die weiteren Sorbenzien miteinander gemischt sind.

17. Kraftfahrzeug (1) mit einer Vorrichtung (5) nach einem der Ansprüche 1 - 16, wobei die Vorrichtung (5) auf Basis eines Belegungszustands des umgrenzten Luftvolumens (3) mit Passagieren ansteuerbar ist, um den Kohlendioxid- und Wassergehalt in dem umgrenzten Luftvolumen (3) unabhängig von dem Belegungszustand in einem vorgegebenen Toleranzfeld zu halten.

18. Verfahren zum Betreiben einer Vorrichtung (5) zum kombinierten Reduzieren des Kohlendioxid- und Wassergehalts in einem umgrenzten Luftvolumen (3), nämlich in einem Fahrgastraum eines Kraftfahrzeugs (1), wobei die Vorrichtung (5) eine erste Sorptionseinheit (6) zum kombinierten Sorbieren von Kohlendioxid und Wasser, eine zweite Sorptionseinheit (7) zum kombinierten Sorbieren von Kohlendioxid und Wasser und eine Luftverteilungseinrichtung (19) zum wechselweisen Schalten der Sorptionseinheiten (6, 7) von einem Sorptionsmodus (M1) in einen Desorptionsmodus (M2) und umgekehrt aufweist, wobei das Verfahren die folgenden Schritte aufweist:
a) Schalten (S1) einer der beiden Sorptionseinheiten (6, 7) in Abhängigkeit von dem Kohlendioxid- und Wassergehalt in dem umgrenzten Luftvolumen (3) mit Hilfe der Luftverteilungseinrichtung (19) in den Sorptionsmodus (M1), in dem von der Sorptionseinheit (6, 7) Kohlendioxid und Wasser aus Rohluft (RO) des umgrenzten Luftvolumens (3) sorbiert wird,
b) Schalten (S2) der anderen der beiden Sorptionseinheiten (6, 7) mit Hilfe der Luftverteilungseinrichtung (19) in den Desorptionsmodus (M2), in dem von der Sorptionseinheit (6, 7) Kohlendioxid und Wasser an zugeführte Regenerationsluft (R1) desorbiert wird, und
c) wechselweises Durchführen (S3) der Schritte a) und b) derart, dass in zumindest einem Betriebszustand eine der beiden Sorptionseinheiten (6, 7) in dem Sorptionsmodus (M1) betrieben wird, während die andere der beiden Sorptionseinheiten (6, 7) in dem Desorptionsmodus (M2) betrieben wird,
wobei der Desorptionsmodus (M2) mit aus einer Umgebung (U) des umgrenzten Luftvolumens (3) entnommener Regenerationsluft (R1), mit aus dem umgrenzten Luftvolumen (3) entnommener Regenerationsluft (R1), unter Vakuum oder durch Rezirkulation durchgeführt wird.
und wobei in diejenige Sorptionseinheit (6, 7), welche in dem Desorptionsmodus (M2) betrieben wird, Wärme (Q) eingebracht wird,
wobei die Vorrichtung (5) zum kombinierten Reduzieren des Kohlendioxid- und Wassergehalts ferner ein Heizelement (11, 11A, 11B, 13, 13A, 13B, 74, 75) zum Einbringen von Wärme (Q) in diejenige Sorptionseinheit (6, 7), die sich in dem Desorptionsmodus (M2) befindet, umfasst, wobei das Heizelement (74, 75) stromaufwärts der Sorptionseinheiten (6, 7) positioniert ist und Wärme in die unbeladene Regenerationsluft (R1) einbringt.

19. Verfahren nach Anspruch 18, wobei in dem umgrenzten Luftvolumen (3) der Kohlendioxid- und Wassergehalt gemessen wird, um die Vorrichtung (5) so anzusteuern, dass der Kohlendioxid- und Wassergehalt in dem umgrenzten Luftvolumen (3) in einem vorgegebenen Toleranzfeld gehalten wird.

20. Verfahren nach Anspruch 19, wobei ein Belegungszustand des umgrenzten Luftvolumens (3) mit Passagieren erfasst wird, um die Vorrichtung (5) so anzusteuern, dass der Kohlendioxid- und Wassergehalt in dem umgrenzten Luftvolumen (3) unabhängig von dem Belegungszustand in dem vorgegebenen Toleranzfeld gehalten wird.

## Claims

1. Apparatus (5) for a combined reduction of carbon dioxide and water content in a delimited air volume (3), in particular in a passenger compartment of a motor vehicle (1), having a first sorption unit (6) for combined sorption of carbon dioxide and water, a second sorption unit (7) for a combined sorption of carbon dioxide and water, wherein the first sorption unit (6) and the second sorption unit (7) each contain a plurality of sorbents (8, 9), wherein the sorption units (6, 7) are each convertible from a sorption mode (M1), in which the sorption units (6, 7) sorb carbon dioxide and water from raw air (RO) of the delimited air volume (3), to a desorption mode (M2), in which the sorption units (6, 7) desorb carbon dioxide and water to supplied regeneration air (R1), and vice versa, and an air distribution device (19), with the aid of which the sorption units (6, 7), depending on the carbon dioxide and water content in the delimited air volume (3), can be switched alternately from the sorption mode (M1) to the desorption mode (M2) and vice versa in such a way that in at least one operating state of the apparatus (5), one of the two sorption units (6, 7) is in the sorption mode (M1), while the other of the two sorption units (6, 7) is in the desorption mode (M2), wherein in the desorption mode (M2) the regeneration air (R1) conveyed through the sorption unit (6, 7), which is in the desorption mode, can be supplied as charged regeneration air (R2) to an environment (U), in particular via a desorption line (40), and wherein the regeneration air (R1) is extractable from the delimited air volume (3) or the environment (U), further comprising a heating element (11, 11A, 11B, 13, 13A, 13B, 74, 75) for introducing heat (Q) into that sorption unit (6, 7) which is in the desorption mode (M2), wherein the heating element (74, 75) is positioned upstream of the sorption units (6, 7) and introduces heat into the uncharged regeneration air (R1).

2. Apparatus according to claim 1, wherein the air distribution device (19) features a plurality of valves (20-23) which are switchable in such a way that, during operation of the apparatus (5), that sorption unit (6, 7) which is in the sorption mode (M1) can be supplied with the raw air (RO) from the delimited air volume (3) in order to remove the carbon dioxide and the water from the raw air (RO), and that sorption unit (6, 7) which is in the desorption mode (M2) can be supplied with the regeneration air (R1) in order to remove the carbon dioxide and the water from the sorption unit (6, 7).

3. Apparatus according to claim 1 or 2, wherein a heating element (11, 11A, 11B, 13, 13A, 13B) is integrated in each sorption unit (6, 7).

4. Apparatus according to claim 1 or 2, wherein the sorption units (6, 7) feature a common heating element (74, 75).

5. Apparatus according to one of the claims 1 to 4, further comprising a regeneration valve (36) featuring a first switching position, in which the regeneration air (R1) can be supplied from an environment (U) of the delimited air volume (3) to the sorption unit (6, 7) in the desorption mode (M2), a second switching position in which the regeneration air (R1) can be supplied from the delimited air volume (3) to the sorption unit (6, 7) in the desorption mode (M2), and in particular a third switching position in which the sorption unit (6, 7) in the desorption mode (M2) can be regenerated under vacuum.

6. Apparatus according to one of the claims 1 to 5, further comprising a blowing means (10) which supplies the raw air (RO) to that sorption unit (6, 7) which is in the sorption mode (M1).

7. Apparatus according to claim 6, wherein the blowing means (10, 51) is part of an air-conditioning system (4).

8. Apparatus according to one of the claims 1 to 7, further comprising a desorption blower means (12) which supplies the regeneration air (R1) to that sorption unit (6, 7) which is in the desorption mode (M2), wherein the desorption blower means (12) is disposed on the pressure side or on the suction side with respect to the sorption units (6, 7).

9. Apparatus according to one of the claims 1 to 8, further comprising at least one bypass line (31) and at least one bypass valve (49) which can be selectively switched from a first switching state, in which the raw air (RO) can be supplied to that sorption unit (6, 7) which is in the sorption mode (M1), into a second switching state, in which the raw air (RO) can be guided back into the delimited air volume (3) with the aid of the bypass line (31) around the sorption units (6, 7), and vice versa.

10. Apparatus according to one of the claims 1 to 9, further comprising a second bypass line (310) and a second bypass valve (490) which can be selectively switched from a first switching state, in which the regeneration air (R1) of that sorption unit (6, 7) which is in the desorption mode (M2), into a second switching state, in which the regeneration air (R1) can be switched into the environments (U) with the aid of the bypass line (310) while bypassing the sorption units (6, 7), and vice versa.

11. Apparatus according to claim 8, wherein a branch (401) is provided in the desorption line (40) downstream of the desorption blower means (12) on the pressure side, said branch opening into a recirculation line (510) which is fluidically connected to an inlet of a regeneration valve (36) or leads downstream of an outlet of the regeneration valve (36) into a regeneration air line (360) connected to the outlet.

12. Apparatus according to claim 11, wherein the branch (401) is switchable by means of a recirculation valve (500), so that by means of the desorption blower means (12) the regeneration air (R1) can be selectively recirculated through the sorption units (6, 7) or directed into the environment (U).

13. Apparatus according to claim 11 or 12, wherein the recirculation line (510) is fluidically connected to the regeneration valve (36), wherein by means of the regeneration valve (36) a fourth switching position, in which the sorption unit (6, 7) is in desorption mode (M2) can be supplied with the regeneration air (R1) from the recirculation line (510), and can be switched vice versa.

14. Apparatus according to claim 11 or 12, wherein a throttle valve, in particular an adjustable throttle valve (501), is fluidically disposed between the outlet of the regeneration valve (36) and the opening of the recirculation line (510) into the regeneration air line (360).

15. Apparatus according to one of the claims 1 to 14, wherein the sorption units (6, 7) are adapted to remove fine particles, nitrogen oxides and/or volatile organic compounds from the raw air (RO) in addition to carbon dioxide and water.

16. Apparatus according to claim 15, wherein the sorption units (6, 7) feature a first sorbent (8) suitable for sorbing carbon dioxide, a second sorbent (9) suitable for sorbing water, and further sorbents suitable for removing fine particles, nitrogen oxides and/or volatile organic compounds from the raw air (RO), wherein the further sorbents are preferably introduced between two carrier layers, in particular of a non-woven fabric, or wherein the sorbents (8, 9) and the further sorbents are combined with each other.

17. Motor vehicle (1) having an apparatus (5) according to one of the claims 1 to 16, wherein the apparatus (5) is controllable based on an occupancy state of the delimited air volume (3) with passengers in order to maintain the carbon dioxide and water content in the delimited air volume (3) within a predetermined margin of tolerance regardless of the occupancy state.

18. Method for operating an apparatus (5) according to claim 1 for a combined reduction of the carbon dioxide and water content in a delimited air volume (3), in particular in a passenger compartment of a motor vehicle (1), wherein the apparatus (5) features a first sorption unit (6) for a combined sorption of carbon dioxide and water, a second sorption unit (7) for a combined sorption of carbon dioxide and water, and an air distribution device (19) for alternately switching the sorption units (6, 7) from a sorption mode (M1) to a desorption mode (M2) and vice versa, wherein the method features the following steps:
a) switching (S1) one of the two sorption units (6, 7) as a function of the carbon dioxide and water content in the delimited air volume (3) with the aid of the air distribution device (19) into the sorption mode (M1) in which carbon dioxide and water are sorbed by the sorption unit (6, 7) from raw air (RO) of the delimited air volume (3),
b) switching (S2) the other of the two sorption units (6, 7) with the aid of the air distribution device (19) into the desorption mode (M2), in which carbon dioxide and water are desorbed from the sorption unit (6, 7) to supplied regeneration air (R1), and
c) alternately carrying out (S3) steps a) and b) in such a way that, in at least one operating state, one of the two sorption units (6, 7) is operated in the sorption mode (M1), while the other of the two sorption units (6, 7) is operated in the desorption mode (M2),
wherein the desorption mode (M2) is carried out with regeneration air (R1) taken from an environment (U) of the delimited air volume (3), with regeneration air (R1) taken from the delimited air volume (3), under vacuum or by recirculation,
and wherein heat (Q) is introduced into that sorption unit (6, 7) which is operated in the desorption mode (M2),
wherein the apparatus (5) for a combined reduction of the carbon dioxide and water content further comprises a heating element (11, 11A, 11B, 13, 13A, 13B, 74, 75) for introducing heat (Q) into that sorption unit (6, 7) which is in the desorption mode (M2), wherein the heating element (74, 75) is positioned upstream of the sorption units (6, 7) and introduces heat into the uncharged regeneration air (R1).

19. Method according to claim 18, wherein in the delimited air volume (3) the carbon dioxide and water content is measured in order to control the apparatus (5) in such a way that the carbon dioxide and the water content in the delimited air volume (3) is kept within a predetermined margin of tolerance.

20. Method according to claim 19, wherein an occupancy state of the delimited air volume (3) having passengers is detected in order to control the apparatus (5) to maintain the carbon dioxide and water content in the delimited air volume (3) within the predetermined margin of tolerance regardless of the occupancy state.

## Revendications

1. Dispositif (5) pour la réduction combinée de la teneur en dioxyde de carbone et en eau dans un volume d'air délimité (3), plus précisément dans un habitacle d'un véhicule automobile (1), ayant une première unité de sorption (6) pour la sorption combinée de dioxyde de carbone et d'eau, une seconde unité de sorption (7) pour la sorption combinée de dioxyde de carbone et d'eau, dans lequel la première unité de sorption (6) et la seconde unité de sorption (7) contiennent chacune une pluralité de sorbants (8, 9), dans lequel les unités de sorption (6, 7) peuvent être amenées respectivement d'un mode de sorption (M1), dans lequel les unités de sorption (6, 7) absorbent le dioxyde de carbone et l'eau de l'air brut (RO) du volume d'air délimité (3), à un mode de désorption (M2), dans lequel les unités de sorption (6, 7) désorbent le dioxyde de carbone et l'eau de l'air de régénération fourni (R1), et vice versa, et un dispositif de distribution d'air (19), à l'aide duquel les unités de sorption (6, 7), en fonction de la teneur en dioxyde de carbone et en eau dans le volume d'air délimité (3), peuvent être commutées en alternance du mode de sorption (M1) au mode de désorption (M2) et vice versa, de telle manière que dans au moins un état opérationnel du dispositif (5), l'une des deux unités de sorption (6, 7) se trouve dans le mode de sorption (M1), alors que l'autre des deux unités de sorption (6, 7) se trouve dans le mode de désorption (M2), dans lequel, dans le mode de désorption (M2), l'air de régénération (R1) conduit à travers l'unité de sorption (6, 7) qui se trouve dans le mode de désorption, peut être amené en tant qu'air de régénération chargé (R2) à un environnement (U), notamment par l'intermédiaire d'une conduite de désorption (40), et dans lequel l'air de régénération (R1) peut être prélevé dans le volume d'air délimité (3) ou dans l'environnement (U), comprenant en outre un élément de chauffage (11, 11A, 11B, 13, 13A, 13B, 74, 75) pour introduire de la chaleur (Q) dans l'unité de sorption (6, 7) qui se trouve dans le mode de désorption (M2), dans lequel l'élément de chauffage (74, 75) est positionné en amont des unités de sorption (6, 7) et introduit de la chaleur dans l'air de régénération non chargé (R1).

2. Dispositif selon la revendication 1, dans lequel le dispositif de distribution d'air (19) présente une pluralité de soupapes (20 - 23) qui peuvent être commutées de telle sorte que, pendant le fonctionnement du dispositif (5), l'air brut (RO) peut être amené à partir du volume d'air délimité (3) à l'unité de sorption (6, 7) qui se trouve dans le mode de sorption (M1), pour éliminer le dioxyde de carbone et l'eau de l'air brut (RO), et à l'unité de sorption (6, 7) qui se trouve dans le mode de désorption (M2), l'air de régénération (R1) peut être amené pour éliminer le dioxyde de carbone et l'eau de l'unité de sorption (6, 7).

3. Dispositif selon la revendication 1 ou 2, dans lequel un élément de chauffage (11, 11A, 11B, 13, 13A, 13B) est intégré dans chaque unité de sorption (6, 7).

4. Dispositif selon la revendication 1 ou 2, dans lequel les unités de sorption (6, 7) présentent un élément de chauffage commun (74, 75).

5. Dispositif selon l'une quelconque des revendications 1 à 4, comprenant en outre une soupape de régénération (36) qui présente une première position de commutation dans laquelle l'air de régénération (R1) peut être amené à l'unité de sorption (6, 7) se trouvant en mode de désorption (M2) à partir d'un environnement (U) du volume d'air délimité (3), une deuxième position de commutation, dans laquelle l'air de régénération (R1) peut être amené à l'unité de sorption (6, 7) se trouvant en mode de désorption (M2) à partir du volume d'air délimité (3), et notamment une troisième position de commutation dans laquelle l'unité de sorption (6, 7) se trouvant en mode de désorption (M2) peut être régénérée sous vide.

6. Dispositif selon l'une quelconque des revendications 1 à 5, comprenant en outre un dispositif de soufflage (10) qui fournit l'air brut (RO) à l'unité de sorption (6, 7) qui se trouve dans le mode de sorption (M1).

7. Dispositif selon la revendication 6, dans lequel le dispositif de soufflage (10, 51) fait partie d'un dispositif de climatisation (4).

8. Dispositif selon l'une quelconque des revendications 1 à 7, comprenant en outre un dispositif de soufflage de désorption (12) qui fournit l'air de régénération (R1) à l'unité de sorption (6, 7) qui se trouve dans le mode de désorption (M2), dans lequel le dispositif de soufflage de désorption (12) est disposé côté pression ou côté aspiration par rapport aux unités de sorption (6, 7).

9. Dispositif selon l'une quelconque des revendications 1 à 8, comprenant en outre au moins une conduite de dérivation (31) et au moins une soupape de dérivation (49) qui peut passer sélectivement d'un premier état de commutation, dans lequel l'air brut (RO) peut être fournit à l'unité de sorption (6, 7) qui se trouve en mode de sorption (M1), dans un deuxième état de commutation dans lequel l'air brut (RO) peut être dirigé à l'aide de la conduite de dérivation (31) autour des unités de sorption (6, 7) en retour dans le volume d'air délimité (3), et vice versa.

10. Dispositif selon l'une quelconque des revendications 1 à 9, comprenant en outre une seconde conduite de dérivation (310) et une seconde soupape de dérivation (490) qui peut passer sélectivement d'un premier état de commutation, dans lequel l'air de régénération (R1) peut être fournit à l'unité de sorption (6, 7) qui se trouve en mode de désorption (M2), dans un second état de commutation, dans lequel l'air de régénération (R1) peut être commuté à l'aide de la conduite de dérivation (310) en contournant les unités de sorption (6, 7) dans environnement (U), et vice versa.

11. Dispositif selon la revendication 8, dans lequel est prévu dans la conduite de désorption (40), en aval du dispositif de soufflage de désorption (12) côté pression, une dérivation (401) qui se termine dans une conduite de recirculation (510) qui est reliée fluidiquement à une entrée d'une soupape de régénération (36) ou qui se termine en aval d'une sortie de la soupape de régénération (36) dans une conduite d'air de régénération (360) raccordée à la sortie.

12. Dispositif selon la revendication 11, dans lequel la dérivation (401) peut être commutée à l'aide d'une soupape de recirculation (500), de sorte qu'à l'aide du dispositif de soufflage de désorption (12), l'air de régénération (R1) peut être sélectivement recirculé à travers les unités de sorption (6, 7) ou dirigé vers l'environnement (U).

13. Dispositif selon la revendication 11 ou 12, dans lequel la conduite de recirculation (510) est raccordée fluidiquement à la soupape de régénération (36), dans lequel une quatrième position de commutation, dans laquelle l'unité de sorption (6, 7) se trouvant en mode de désorption (M2) peut être alimentée en air de régénération (R1) à partir de la conduite de recirculation (510), et vice versa, peut être commutée au moyen de la soupape de régénération (36).

14. Dispositif selon la revendication 11 ou 12, dans lequel une soupape d'étranglement, notamment une soupape d'étranglement ajustable (501), est disposée fluidiquement entre la sortie de la soupape de régénération (36) et l'embouchure de la conduite de recirculation (510) dans la conduite d'air de régénération (360).

15. Dispositif selon l'une quelconque des revendications 1 à 14, dans lequel les unités de sorption (6, 7) sont aptes à extraire de l'air brut (RO), outre le dioxyde de carbone et l'eau, les particules fines, les oxydes d'azote et/ou les composés organiques volatils.

16. Dispositif selon la revendication 15, dans lequel les unités de sorption (6, 7) présentent un premier sorbant (8) apte à absorber le dioxyde de carbone, un deuxième sorbant (9) apte à absorber l'eau et d'autres sorbants aptes à extraire de l'air brut (RO) des particules fines, des oxydes d'azote et/ou des composés organiques volatils, dans lequel les autres sorbants sont de préférence introduits entre deux couches de support, notamment en un non-tissé, ou dans lequel les sorbants (8, 9) et les autres sorbants sont mélangés entre eux.

17. Véhicule automobile (1) ayant un dispositif (5) selon l'une quelconque des revendications 1 à 16, dans lequel le dispositif (5) peut être commandé sur la base d'un état d'occupation du volume d'air délimité (3) par des passagers, afin de maintenir la teneur en dioxyde de carbone et en eau dans le volume d'air délimité (3) dans une plage de tolérance prédéterminée, indépendamment de l'état d'occupation.

18. Procédé de fonctionnement d'un dispositif (5) selon la revendication 1 pour la réduction combinée de la teneur en dioxyde de carbone et en eau dans un volume d'air délimité (3), plus précisément dans un habitacle d'un véhicule automobile (1), dans lequel le dispositif (5) comprend une première unité de sorption (6) pour la sorption combinée de dioxyde de carbone et d'eau, une seconde unité de sorption (7) pour la sorption combinée de dioxyde de carbone et d'eau et un dispositif de distribution d'air (19) pour commuter alternativement les unités de sorption (6, 7) d'un mode de sorption (M1) à un mode de désorption (M2) et vice versa, dans lequel le procédé comprend les étapes suivantes :
a) commutation (S1) de l'une des deux unités de sorption (6, 7) en fonction de la teneur en dioxyde de carbone et en eau dans le volume d'air délimité (3) à l'aide du dispositif de distribution d'air (19) dans le mode de sorption (M1) dans lequel le dioxyde de carbone et l'eau sont absorbés de l'air brut (RO) du volume d'air délimité (3) par l'unité de sorption (6, 7),
b) commutation (S2) de l'autre des deux unités de sorption (6, 7) à l'aide du dispositif de distribution d'air (19) dans le mode de désorption (M2) dans lequel le dioxyde de carbone et l'eau sont désorbés par l'unité de sorption (6, 7) à de l'air de régénération fourni (R1), et
c) l'exécution alternée (S3) des étapes a) et b) de telle sorte que, dans au moins un état de fonctionnement, l'une des deux unités de sorption (6, 7) est exploitée dans le mode de sorption (M1), alors que l'autre des deux unités de sorption (6, 7) est exploitée dans le mode de désorption (M2),
dans lequel le mode de désorption (M2) est effectué avec de l'air de régénération (R1) provenant d'un environnement (U) du volume d'air délimité (3), avec de l'air de régénération (R1) provenant du volume d'air délimité (3), sous vide ou par recirculation,
et dans lequel de la chaleur (Q) est introduite dans l'unité de sorption (6, 7) qui fonctionne dans le mode de désorption (M2),
dans lequel le dispositif (5) pour réduire de manière combinée la teneur en dioxyde de carbone et en eau comprend en outre un élément de chauffage (11, 11A, 11B, 13, 13A, 13B, 74, 75) pour introduire de la chaleur (Q) dans l'unité de sorption (6, 7) qui se trouve dans le mode de désorption (M2), dans lequel l'élément de chauffage (74, 75) est positionné en amont des unités de sorption (6, 7) et introduit de la chaleur dans l'air de régénération non chargé (R1).

19. Procédé selon la revendication 18, dans lequel est mesuré dans le volume d'air délimité (3) la teneur en dioxyde de carbone et en eau afin de commander le dispositif (5) pour maintenir la teneur en dioxyde de carbone et en eau dans le volume d'air délimité (3) dans une plage de tolérance prédéterminée.

20. Procédé selon la revendication 19, dans lequel un état d'occupation du volume d'air délimité (3) par des passagers est détecté pour commander le dispositif (5) de manière à maintenir la teneur en dioxyde de carbone et en eau dans le volume d'air délimité (3) dans la plage de tolérance prédéterminée indépendamment de l'état d'occupation.
